# EUROPEAN PATENT SPECIFICATION

(11) **EP 1 693 456 B1**
(45) Date of publication and mention of the grant of the patent: **19.01.2011**
(21) Application number: 04818967.4
(22) Date of filing: 19.11.2004
(51) Int. Cl.: C12N 15/12, C12P 21/02, C07K 14/705, C07K 16/18, C12N 1/15, C12N 1/19, C12N 1/21, C12N 5/10, A61K 38/17, A61K 31/7088, A61K 39/395, A61P 25/28, A61P 25/22, A61P 9/10, A61P 3/04, A61P 3/10, A61P 35/00, G01N 33/53

(54) **METHOD OF IDENTIFYING MODULATORS OF A G PROTEIN-COUPLED RECEPTOR**
VERFAHREN ZUR IDENTIFIZIERUNG VON MODULATOREN EINES G-PROTEIN-GEKOPPELTEN REZEPTORS
PROCEDE D'IDENTIFICATION DE MODULATEURS D'UN RECEPTEUR COUPLE A LA PROTEINE G

(30) Priority: 19.11.2003 JP 2003389624; 26.04.2004 JP 2004130371; 19.10.2004 JP 2004304780
(43) Date of publication of application: 23.08.2006
(73) Proprietor: Daiichi Sankyo Company, Limited, Chuo-ku Tokyo 103-8426 (JP); Kazusa DNA Research Institute Foundation, Kisarazu-shi, Chiba 292-0818 (JP)
(72) Inventor: OHARA, Osamu, c/o KAZUSA DNA RES. INST. FOUND., Kisarazu-shi, Chiba 2920818 (JP); NAGASE, Takahiro, c/o KAZUSA DNA RES. INST. FOUND., Kisarazu-shi, Chiba 2920818 (JP); OHISHI, Michio, c/o KAZUSA DNA RES. INST. FOUND., Kisarazu-shi, Chiba 2920818 (JP); OKAJIMA, Daisuke, DAIICHI PHARMACEUTICAL CO., LTD. TOKYO R&D CENTER,, Tokyo 134-8630 (JP); YOKOTA, Hiroshi, DAIICHI PHARMACEUTICAL CO., LTD. TOKYO R&D CENTER,, Tokyo 134-8630 (JP)
(74) Representative: Zwicker, Jörk
(86) International application number: PCT/JP2004/017239
(87) International publication number: WO 2005/049833

(56) References cited:
- JP-A- 11 032 766
- JP-A- 2003 284 573
- DATABASE Geneseq [Online] 1 January 2004 (2004-01-01), "Human GPCR protein SEQ ID NO:870." XP002435010 retrieved from EBI accession no. GSP:ADC86417 Database accession no. ADC86417 -& EP 1 270 724 A (NAT INST OF ADVANCED IND SCIEN [JP]; CT FOR ADVANCED SCIENCE & TECH [J) 2 January 2003 (2003-01-02)
- KEE HAE JIN ET AL: "Expression of brain-specific angiogenesis inhibitor 2 (BAI2) in normal and ischemic brain: Involvement of BAI2 in the ischemia-induced brain angiogenesis" JOURNAL OF CEREBRAL BLOOD FLOW AND METABOLISM, vol. 22, no. 9, September 2002 (2002-09), pages 1054-1067, XP002434909 ISSN: 0271-678X -& DATABASE UniProt [Online] 11 July 2006 (2006-07-11), "Brain-specific angiogenesis inhibitor 2 precursor." XP002435011 retrieved from EBI accession no. UNIPROT:Q8CGM1 Database accession no. Q8CGM1
- DATABASE Geneseq [Online] 4 March 2003 (2003-03-04), "Human brain-specific angiogenesis inhibitor 2 protein SEQ ID NO:344." XP002435012 retrieved from EBI accession no. GSP:ABP81929 Database accession no. ABP81929 -& WO 02/061087 A (LIFESPAN BIOSCIENCES INC [US]; BURMER GLENNA C [US]; ROUSH CHRISTINE L) 8 August 2002 (2002-08-08)
- NISHIMORI H ET AL: "NOVEL BRAIN-SPECIFIC P53-TARGET GENE, BAI1, CONTAINING THROMBOSPONDIN 1 REPEATS INHIBITS EXPERIMENTAL ANGIOGENESIS" ONCOGENE, BASINGSTOKE, HANTS, GB, vol. 15, no. 18, October 1997 (1997-10), pages 2145-2150, XP000923392 ISSN: 0950-9232
- KAUR BALVEEN ET AL: "Brain angiogenesis inhibitor 1 is differentially expressed in normal brain and glioblastoma independently of p53 expression." AMERICAN JOURNAL OF PATHOLOGY, vol. 162, no. 1, January 2003 (2003-01), pages 19-27, XP002434910 ISSN: 0002-9440
- VAN BREE L ET AL: "Homolateral cerebrocortical changes in neuropeptide and receptor expression after minimal cortical infarction" NEUROSCIENCE, vol. 69, no. 3, 1995, pages 847-858, XP002435002 ISSN: 0306-4522
- SHIRATSUCHI T. ET AL: 'Cloning and characterization of BAI2 and BAI3, novel genes homologous to brain-specific angiogenesis' CYTOGENET. CELL GENET. vol. 79, 1997, pages 103 - 108, XP002983770
- DUNLOP J. ET AL: 'Full and partial agonist activity of C-terminal cholecystokinin peptides at the cloned human CCK-A receptor expressed in Chinese hamster ovary cells' PEPTIDES vol. 18, no. 6, 1997, pages 865 - 868, XP002983771

### SEQUENCE LISTING

<110> DAIICHI PHARMACEUTICAL C0., LTD. KAZUSA DNA RESEARCH INSTITUTE FOUNDATION
<120> A gene coding for G-protein coupled receptor and the gene product of the same
<130> GP04-1020PCT
<150> JP P2003-389624
   <151> 2003-11-19
<150> JP P2004-130371
   <151> 2004-04-26
<150> JP P2004-304780
   <151> 2004-10-19
<160> 14
<170> PatentIn version 3.1
<210> 1
   <211> 4557
   <212> DNA
   <213> homo sapiens
<220>
   <221> misc_feature
   <223> DNA coding for a novel functional membrane protein receptor
<400> 1
   atgaccccag cctgtcccct cttactgtct gtgattctgt ccctgcgcct ggccaccgcc 60
   ttcgaccccg cccccagtgc ctgctctgcc ctggcctcgg gtgtgctcta cggggccttc 120
   tcgctgcagg acctctttcc taccatcgcc tcgggctgct cctggaccct ggagaaccct 180
   gaccccacca agtactccct ctacctgcgc ttcaaccgcc aggagcaggt gtgcgcacac 240
   tttgcccccc gcctgctgcc cctggaccac tacctggtca actttacctg cctgcggcct 300
   agccccgagg aggcggtggc ccaggcggag tcagaggtgg ggcggccaga agaggaggag 360
   gcagaggcgg cagcggggtt ggagctgtgc agcggctcag gcccctttac cttcctgcac 420
   ttcgacaaga acttcgtgca gctgtgcctg tcggctgagc cctccgaggc cccgcgcctg 480
   ctggcgcccg ctgccctagc cttccgcttt gtcgaggtct tgctcatcaa caacaacaac 540
   tctagccaat tcacctgtgg tgtgctctgc cgctggagtg aggagtgtgg ccgcgctgcc 600
   ggcagggcct gcggctttgc tcagccaggc tgcagctgcc ctggagaggc gggggccggc 660
   tccaccacca ccacatctcc aggccctcct gctgcccaca ccctgtccaa tgccctggtg 720
   cccgggggcc cagccccacc tgctgaggcc gatttgcact cggggagcag caatgatctg 780
   ttcacaaccg agatgagata tggtgaggag ccggaagagg aaccgaaagt gaaaacccag 840
   tggccgaggt ctgcagatga gcctgggcta tacatggcgc agacagtgca cggcgtgtgg 900
   gaggagtggg ggtcctggag cctgtgctcc cgcagctgcg ggcgggggtc ccggagccgg 960
   atgcggacct gcgtgccccc ccagcacggc ggcaaggcct gcgagggtcc tgagctgcag 1020
   actaagctct gcagtatggc tgcctgcccg gtggaaggcc agtggttaga atggggtccc 1080
   tggggcccat gctccacgtc ctgtgccaat gggacccaac agcgcagccg gaagtgcagc 1140
   gtggcgggcc cagcctgggc cacatgcacg ggtgccctca ctgacacccg ggagtgcagc 1200
   aacctcgagt gcccggccac tgatagcaag tgggggccat ggaatgcgtg gagcctgtgc 1260
   tctaagacgt gtgacacagg ctggcagcgc cgcttccgca tgtgccaggc cacgggcacg 1320
   cagggctacc cctgcgaggg caccggagag gaggtgaagc cttgtagtga gaagaggtgt 1380
   ccagccttcc atgagatgtg cagggatgag tacgtgatgc tgatgacgtg gaagaaggca 1440
   gctgctggcg agatcatcta caacaagtgc cccccgaatg cctcagggtc tgccagccgc 1500
   cgctgtctcc tcagtgccca aggcgtggcg tactgggggc tgcccagctt tgctcgctgc 1560
   atctcccatg agtaccgcta cctgtatctg tcacttaggg agcacctggc caaggggcag 1620
   cgcatgctgg caggcgaggg catgtcgcag gtggtgcgca gcctgcagga gctactggcc 1680
   cggcgcacct actatagtgg ggacctgctc ttctctgtgg acattctgag gaatgtcact 1740
   gacaccttta agagggccac ctacgtgccc tcggctgatg atgtgcagcg cttcttccag 1800
   gtggtgagct tcatggtgga tgcggaaaac aaggagaagt gggacgatgc tcagcaggtg 1860
   tcccctggct ctgtgcacct gctccgtgtc gtggaggact tcattcacct ggtgggcgat 1920
   gctctcaagg ccttccagag ctctctgatt gtcacagata atctagtgat cagcattcag 1980
   cgagagcccg tctcagctgt gtccagtgac atcacgttcc ccatgcgggg ccgccggggc 2040
   atgaaggact gggtgcggca ctcagaggac cgcctcttcc tgcccaagga ggtgctcagc 2100
   ctctcctccc cagggaagcc agccacatct ggggcagcag gcagccctgg cagggggagg 2160
   ggcccaggaa cggtgcctcc tggcccaggc cactcccacc agcgcctcct cccagcagac 2220
   cctgatgagt cctcctactt tgtgatcggt gctgtactct accgcaccct tggcctcatc 2280
   ctgccgcctc ccaggccccc gctggccgtc acatcccggg tgatgacagt gactgtgcgc 2340
   ccccctaccc agcctccagc tgagcccctc atcactgtgg agctctccta catcatcaat 2400
   gggaccacgg atccccattg cgccagctgg gactactcca gagcagatgc cagctcagga 2460
   gactgggaca ctgaaaattg ccagaccctg gagacccagg cagctcacac ccgctgccag 2520
   tgccagcacc tgtccacctt tgctgtacta gcccagccgc ccaaggacct gaccctggag 2580
   ctggcgggct ccccctcggt ccccctggtg atcggctgtg cagtgtcgtg catggcgctg 2640
   ctcaccctgc tcgccatcta tgccgccttt tggaggttca taaaatctga acgctccatc 2700
   atcttgctga acttctgcct gtccatcttg gcatccaaca tcctgatcct cgtgggccag 2760
   tcccgggtgc tgagcaaggg cgtgtgcacc atgacggctg ccttcctgca cttcttcttt 2820
   ctctcctcct tttgctgggt gcttaccgag gcctggcagt cctacctggc tgtcattggg 2880
   cggatgcgca cccgcctcgt tcgcaagcgc ttcctctgcc tgggctgggg tctgcctgcc 2940
   ctggtggtgg ccgtgtctgt tggctttacc cgaacgaaag gatacggtac atccagctac 3000
   tgctggctct ccctggaggg cggcctgctc tacgcctttg tgggccctgc agccgtcatt 3060
   gtcctggtga acatgctcat cggaatcatc gtcttcaaca agctcatggc acgtgatggc 3120
   atctccgaca aatccaagaa gcagagggcc gggtcggagc ggtgcccctg ggccagcctg 3180
   ctcctcccct gctcagcgtg tggagcggtc cccagccccc tgctcagctc agcctcggcc 3240
   aggaacgcca tggcctcact ctggagctcc tgcgtggtgc tgcccctgct ggcgctcacc 3300
   tggatgtctg ccgtcctggc tatgacagac cgccgttccg tcctcttcca ggccctcttt 3360
   gctgtcttca actccgcgca gggctttgtc atcactgctg tgcactgctt cctgcgccga 3420
   gaggtccagg atgtggtgaa gtgccagatg ggggtgtgcc gggctgatga gagcgaagac 3480
   tcccctgact cgtgtaagaa cgggcagctg cagatcctgt cagactttga aaaggatgtg 3540
   gatctggctt gtcaaacagt gctgttcaag gaggtcaaca cttgcaaccc gtccaccatc 3600
   acgggcacac tatcccgcct gtccctggat gaggatgagg agcccaagtc ctgcctcgtg 3660
   ggccctgagg gcagcctcag cttctcacca ctgcctggga atatcctggt gcccatggca 3720
   gcctcaccag ggctggggga gcctccgccc ccacaggagg ccaaccctgt ttacatgtgt 3780
   ggggagggtg gcctgcggca gctggacctc acatggctgc ggcccactga gccaggctct 3840
   gagggagact acatggtgct gccccggcgg actttgagcc tgcagcctgg cggtgggggt 3900
   ggaggtggtg aggatgcccc cagggcccgg ccggagggga ccccccggcg agctgccaag 3960
   acagtggccc acactgaagg ctaccccagc ttcctgtccg tggaccactc gggcctgggg 4020
   ctgggccctg cctatggatc tctccagaat ccctatggaa tgaccttcca accgccaccg 4080
   ccgacaccca gcgcccgcca agtgcccgag ccaggggagc gcagccggac catgcctcgc 4140
   accgtgcccg gctctaccat gaagatgggc tccctggagc gaaagaaatt acggtattca 4200
   gacctggact ttgagaaggt gatgcacacc cggaaacggc attcagaact ctaccacgag 4260
   ctcaaccaga agttccacac tttcgaccgc taccgcagcc agtccacggc caagagggag 4320
   aagcggtgga gtgtgtcctc gggtggggca gccgagcgga gcgtgtgcac cgataagccc 4380
   agccctgggg agcgccccag cttgtcccaa catcggcgcc atcagagctg gagcaccttc 4440
   aaatctatga cactgggctc gctgcccccc aagccccgag aacggctgac tctgcaccgg 4500
   gcagcagcct gggagcccac agaaccaccg gatggtgact tccagacaga ggtgtga 4557
<210> 2
   <211> 1518
   <212> PRT
   <213> homo sapiens
<220>
   <221> MISC_FEATURE
   <223> Protein that is coded for by the DNA having a nucleotide sequence of SEQ ID NO:1
<220>
   <221> DOMAIN
   <222> (297).. (350)
   <223> TSP-I domain
<220>
   <221> DOMAIN
   <222> (352).. (405)
   <223> TSP-I domain
<220>
   <221> DOMAIN
   <222> (408) .. (461)
   <223> TSP-I domain
<220>
   <221> TRANSMEM
   <222> (870).. (890)
   <223> transmembrane domain
<220>
   <221> TRANSMEM
   <222> (899) .. (919)
   <223> transmembrane domain
<220>
   <221> TRANSMEM
   <222> (928)..(948)
   <223> transmembrane domain
<220>
   <221> TRANSMEM
   <222> (970)..(990)
   <223> transmembrane domain
<220>
   <221> TRANSMEM
   <222> (1012).. (1032)
   <223> transmembrane domain
<220>
   <221> TRANSMEM
   <222> (1087) .. (1107)
   <223> transmembrane domain
<220>
   <221> TRANSMEM
   <222> (1114).. (1134)
   <223> transmembrane domain
<400> 2
   Met Thr Pro Ala Cys Pro Leu Leu Leu Ser Val Ile Leu Ser Leu Arg
   1               5                   10                  15
   Leu Ala Thr Ala Phe Asp Pro Ala Pro Ser Ala Cys Ser Ala Leu Ala
               20                  25                  30
   Ser Gly Val Leu Tyr Gly Ala Phe Ser Leu Gln Asp Leu Phe Pro Thr
   35 40 45
   Ile Ala Ser Gly Cys Ser Trp Thr Leu Glu Asn Pro Asp Pro Thr Lys
   50 55 60
   Tyr Ser Leu Tyr Leu Arg Phe Asn Arg Gln Glu Gln Val Cys Ala His
   65 70 75 80
   Phe Ala Pro Arg Leu Leu Pro Leu Asp His Tyr Leu Val Asn Phe Thr
   85 90 95
   Cys Leu Arg Pro Ser Pro Glu Glu Ala Val Ala Gln Ala Glu Ser Glu
   100 105 110
   Val Gly Arg Pro Glu Glu Glu Glu Ala Glu Ala Ala Ala Gly Leu Glu
   115 120 125
   Leu Cys Ser Gly Ser Gly Pro Phe Thr Phe Leu His Phe Asp Lys Asn
   130 135 140
   Phe Val Gln Leu Cys Leu Ser Ala Glu Pro Ser Glu Ala Pro Arg Leu
   145 150 155 160
   Leu Ala Pro Ala Ala Leu Ala Phe Arg Phe Val Glu Val Leu Leu Ile
   165 170 175
   Asn Asn Asn Asn Ser Ser Gln Phe Thr Cys Gly Val Leu Cys Arg Trp
   180 185 190
   Ser Glu Glu Cys Gly Arg Ala Ala Gly Arg Ala Cys Gly Phe Ala Gln
   195 200 205
   Pro Gly Cys Ser Cys Pro Gly Glu Ala Gly Ala Gly Ser Thr Thr Thr
   210 215 220
   Thr Ser Pro Gly Pro Pro Ala Ala His Thr Leu Ser Asn Ala Leu Val
   225 230 235 240
   Pro Gly Gly Pro Ala Pro Pro Ala Glu Ala Asp Leu His Ser Gly Ser
   245 250 255
   Ser Asn Asp Leu Phe Thr Thr Glu Met Arg Tyr Gly Glu Glu Pro Glu
   260 265 270
   Glu Glu Pro Lys Val Lys Thr Gln Trp Pro Arg Ser Ala Asp Glu Pro
   275 280 285
   Gly Leu Tyr Met Ala Gln Thr Val His Gly Val Trp Glu Glu Trp Gly
   290 295 300
   Ser Trp Ser Leu Cys Ser Arg Ser Cys Gly Arg Gly Ser Arg Ser Arg
   305 310 315 320
   Met Arg Thr Cys Val Pro Pro Gln His Gly Gly Lys Ala Cys Glu Gly
   325 330 335
   Pro Glu Leu Gln Thr Lys Leu Cys Ser Met Ala Ala Cys Pro Val Glu
   340 345 350
   Gly Gln Trp Leu Glu Trp Gly Pro Trp Gly Pro Cys Ser Thr Ser Cys
   355 360 365
   Ala Asn Gly Thr Gln Gln Arg Ser Arg Lys Cys Ser Val Ala Gly Pro
   370 375 380
   Ala Trp Ala Thr Cys Thr Gly Ala Leu Thr Asp Thr Arg Glu Cys Ser
   385 390 395 400
   Asn Leu Glu Cys Pro Ala Thr Asp Ser Lys Trp Gly Pro Trp Asn Ala
   405 410 415
   Trp Ser Leu Cys Ser Lys Thr Cys Asp Thr Gly Trp Gln Arg Arg Phe
   420 425 430
   Arg Met Cys Gln Ala Thr Gly Thr Gln Gly Tyr Pro Cys Glu Gly Thr
   435 440 445
   Gly Glu Glu Val Lys Pro Cys Ser Glu Lys Arg Cys Pro Ala Phe His
   450 455 460
   Glu Met Cys Arg Asp Glu Tyr Val Met Leu Met Thr Trp Lys Lys Ala
   465 470 475 480
   Ala Ala Gly Glu Ile Ile Tyr Asn Lys Cys Pro Pro Asn Ala Ser Gly
   485 490 495
   Ser Ala Ser Arg Arg Cys Leu Leu Ser Ala Gln Gly Val Ala Tyr Trp
   500 505 510
   Gly Leu Pro Ser Phe Ala Arg Cys Ile Ser His Glu Tyr Arg Tyr Leu
   515 520 525
   Tyr Leu Ser Leu Arg Glu His Leu Ala Lys Gly Gln Arg Met Leu Ala
   530 535 540
   Gly Glu Gly Met Ser Gln Val Val Arg Ser Leu Gln Glu Leu Leu Ala
   545 550 555 560
   Arg Arg Thr Tyr Tyr Ser Gly Asp Leu Leu Phe Ser Val Asp Ile Leu
   565 570 575
   Arg Asn Val Thr Asp Thr Phe Lys Arg Ala Thr Tyr Val Pro Ser Ala
   580 585 590
   Asp Asp Val Gln Arg Phe Phe Gln Val Val Ser Phe Met Val Asp Ala
   595 600 605
   Glu Asn Lys Glu Lys Trp Asp Asp Ala Gln Gln Val Ser Pro Gly Ser
   610 615 620
   Val His Leu Leu Arg Val Val Glu Asp Phe Ile His Leu Val Gly Asp
   625 630 635 640
   Ala Leu Lys Ala Phe Gln Ser Ser Leu Ile Val Thr Asp Asn Leu Val
   645 650 655
   Ile Ser Ile Gln Arg Glu Pro Val Ser Ala Val Ser Ser Asp Ile Thr
   660 665 670
   Phe Pro Met Arg Gly Arg Arg Gly Met Lys Asp Trp Val Arg His Ser
   675 680 685
   Glu Asp Arg Leu Phe Leu Pro Lys Glu Val Leu Ser Leu Ser Ser Pro
   690 695 700
   Gly Lys Pro Ala Thr Ser Gly Ala Ala Gly Ser Pro Gly Arg Gly Arg
   705 710 715 720
   Gly Pro Gly Thr Val Pro Pro Gly Pro Gly His Ser His Gln Arg Leu
   725 730 735
   Leu Pro Ala Asp Pro Asp Glu Ser Ser Tyr Phe Val Ile Gly Ala Val
   740 745 750
   Leu Tyr Arg Thr Leu Gly Leu Ile Leu Pro Pro Pro Arg Pro Pro Leu
   755 760 765
   Ala Val Thr Ser Arg Val Met Thr Val Thr Val Arg Pro Pro Thr Gln
   770 775 780
   Pro Pro Ala Glu Pro Leu Ile Thr Val Glu Leu Ser Tyr Ile Ile Asn
   785 790 795 800
   Gly Thr Thr Asp Pro His Cys Ala Ser Trp Asp Tyr Ser Arg Ala Asp
   805 810 815
   Ala Ser Ser Gly Asp Trp Asp Thr Glu Asn Cys Gln Thr Leu Glu Thr
   820 825 830
   Gln Ala Ala His Thr Arg Cys Gln Cys Gln His Leu Ser Thr Phe Ala
   835 840 845
   Val Leu Ala Gln Pro Pro Lys Asp Leu Thr Leu Glu Leu Ala Gly Ser
   850 855 860
   Pro Ser Val Pro Leu Val Ile Gly Cys Ala Val Ser Cys Met Ala Leu
   865 870 875 880
   Leu Thr Leu Leu Ala Ile Tyr Ala Ala Phe Trp Arg Phe Ile Lys Ser
   885 890 895
   Glu Arg Ser Ile Ile Leu Leu Asn Phe Cys Leu Ser Ile Leu Ala Ser
   900 905 910
   Asn Ile Leu Ile Leu Val Gly Gln Ser Arg Val Leu Ser Lys Gly Val
   915 920 925
   Cys Thr Met Thr Ala Ala Phe Leu His Phe Phe Phe Leu Ser Ser Phe
   930 935 940
   Cys Trp Val Leu Thr Glu Ala Trp Gln Ser Tyr Leu Ala Val Ile Gly
   945 950 955 960
   Arg Met Arg Thr Arg Leu Val Arg Lys Arg Phe Leu Cys Leu Gly Trp
   965 970 975
   Gly Leu Pro Ala Leu Val Val Ala Val Ser Val Gly Phe Thr Arg Thr
   980 985 990
   Lys Gly Tyr Gly Thr Ser Ser Tyr Cys Trp Leu Ser Leu Glu Gly Gly
   995 1000 1005
   Leu Leu Tyr Ala Phe Val Gly Pro Ala Ala Val Ile Val Leu Val
   1010 1015 1020
   Asn Met Leu Ile Gly Ile Ile Val Phe Asn Lys Leu Met Ala Arg
   1025 1030 1035
   Asp Gly Ile Ser Asp Lys Ser Lys Lys Gln Arg Ala Gly Ser Glu
   1040 1045 1050
   Arg Cys Pro Trp Ala Ser Leu Leu Leu Pro Cys Ser Ala Cys Gly
   1055 1060 1065
   Ala Val Pro Ser Pro Leu Leu Ser Ser Ala Ser Ala Arg Asn Ala
   1070 1075 1080
   Met Ala Ser Leu Trp Ser Ser Cys Val Val Leu Pro Leu Leu Ala
   1085 1090 1095
   Leu Thr Trp Met Ser Ala Val Leu Ala Met Thr Asp Arg Arg Ser
   1100 1105 1110
   Val Leu Phe Gln Ala Leu Phe Ala Val Phe Asn Ser Ala Gln Gly
   1115 1120 1125
   Phe Val Ile Thr Ala Val His Cys Phe Leu Arg Arg Glu Val Gln
   1130 1135 1140
   Asp Val Val Lys Cys Gln Met Gly Val Cys Arg Ala Asp Glu Ser
   1145 1150 1155
   Glu Asp Ser Pro Asp Ser Cys Lys Asn Gly Gln Leu Gln Ile Leu
   1160 1165 1170
   Ser Asp Phe Glu Lys Asp Val Asp Leu Ala Cys Gln Thr Val Leu
   1175 1180 1185
   Phe Lys Glu Val Asn Thr Cys Asn Pro Ser Thr Ile Thr Gly Thr
   1190 1195 1200
   Leu Ser Arg Leu Ser Leu Asp Glu Asp Glu Glu Pro Lys Ser Cys
   1205 1210 1215
   Leu Val Gly Pro Glu Gly Ser Leu Ser Phe Ser Pro Leu Pro Gly
   1220 1225 1230
   Asn Ile Leu Val Pro Met Ala Ala Ser Pro Gly Leu Gly Glu Pro
   1235 1240 1245
   Pro Pro Pro Gln Glu Ala Asn Pro Val Tyr Met Cys Gly Glu Gly
   1250 1255 1260
   Gly Leu Arg Gln Leu Asp Leu Thr Trp Leu Arg Pro Thr Glu Pro
   1265 1270 1275
   Gly Ser Glu Gly Asp Tyr Met Val Leu Pro Arg Arg Thr Leu Ser
   1280 1285 1290
   Leu Gln Pro Gly Gly Gly Gly Gly Gly Gly Glu Asp Ala Pro Arg
   1295 1300 1305
   Ala Arg Pro Glu Gly Thr Pro Arg Arg Ala Ala Lys Thr Val Ala
   1310 1315 1320
   His Thr Glu Gly Tyr Pro Ser Phe Leu Ser Val Asp His Ser Gly
   1325 1330 1335
   Leu Gly Leu Gly Pro Ala Tyr Gly Ser Leu Gln Asn Pro Tyr Gly
   1340 1345 1350
   Met Thr Phe Gln Pro Pro Pro Pro Thr Pro Ser Ala Arg Gln Val
   1355 1360 1365
   Pro Glu Pro Gly Glu Arg Ser Arg Thr Met Pro Arg Thr Val Pro
   1370 1375 1380
   Gly Ser Thr Met Lys Met Gly Ser Leu Glu Arg Lys Lys Leu Arg
   1385 1390 1395
   Tyr Ser Asp Leu Asp Phe Glu Lys Val Met His Thr Arg Lys Arg
   1400 1405 1410
   His Ser Glu Leu Tyr His Glu Leu Asn Gln Lys Phe His Thr Phe
   1415 1420 1425
   Asp Arg Tyr Arg Ser Gln Ser Thr Ala Lys Arg Glu Lys Arg Trp
   1430 1435 1440
   Ser Val Ser Ser Gly Gly Ala Ala Glu Arg Ser Val Cys Thr Asp
   1445 1450 1455
   Lys Pro Ser Pro Gly Glu Arg Pro Ser Leu Ser Gln His Arg Arg
   1460 1465 1470
   His Gln Ser Trp Ser Thr Phe Lys Ser Met Thr Leu Gly Ser Leu
   1475 1480 1485
   Pro Pro Lys Pro Arg Glu Arg Leu Thr Leu His Arg Ala Ala Ala
   1490 1495 1500
   Trp Glu Pro Thr Glu Pro Pro Asp Gly Asp Phe Gln Thr Glu Val
   1505 1510 1515
<210> 3
   <211> 4
   <212> PRT
   <213> homo sapiens
<220>
   <221> MISC_FEATURE
   <222> (1).. (4)
   <223> Partial amino acid sequence which exists in each C -terminal regi on of the peptide consisting of the amino acid sequence of SEQ ID NO:2, human BAI1 and human BAI2,
<400> 3
   Gln Thr Glu Val
   1
<210> 4
   <211> 28
   <212> DNA
   <213> Artificial
<220>
   <223> Designed oligonucleotide for using as a primer
<400> 4
   ggggacaagt ttgtacaaaa aagcaggc 28
<210> 5
   <211> 28
   <212> DNA
   <213> Artificial
<220>
   <223> Designed oligonucleotide for using as a primer
<400> 5
   ggggaccact ttgtacaaga aagctggg 28
<210> 6
   <211> 31
   <212> DNA
   <213> Artificial
<220>
   <223> Designed oligonucleotide for using as a primer
<400> 6
   aaaaagcagg ctggatgacc ccagcctgtc c 31
<210> 7
   <211> 32
   <212> DNA
   <213> Artificial
<220>
   <223> Designed oligonucleotide for using as a primer
<400> 7
   agaaagctgg gtgcactcac acctctgtct gg 32
<210> 8
   <211> 36
   <212> DNA
   <213> Artificial
<220>
   <223> Designed oligonucleotide for using as a primer
<400> 8
   tacaaaaaag caggcttcga ccccgccccc agtgcc 36
<210> 9
   <211> 33
   <212> DNA
   <213> artificial
<220>
   <223> Designed oligonucleotide for using as a primer
<400> 9
   ggggtcgaag cctgcttttt tgtacaaagt tgg 33
<210> 10
   <211> 25
   <212> DNA
   <213> Artificial
<220>
   <223> Designed oligonucleotide for using as a primer
<400> 10
   tcgcgttaac gctagcatgg atctc 25
<210> 11
   <211> 24
   <212> DNA
   <213> Artificial
<220>
   <223> Designed oligonucleotide for using as a primer
<400> 11
   gtaacatcag agattttgag acac 24
<210> 12
   <211> 125
   <212> DNA
   <213> Artificial
<220>
   <223> Synthesized oligonucleotide
<400> 12
   gggggaattc accatggcct taccagtgac cgccttgctc ctgccgctgg ccttgctgct 60
   ccacgccgcc aggccggatt acgcttctta cccatacgat gttccagatt acgctgtcga 120
   cgggg 125
<210> 13
   <211> 125
   <212> DNA
   <213> Artificial
<220>
   <223> Synthesized oligonucleotide
<400> 13
   ccccgtcgac agcgtaatct ggaacatcgt atgggtaaga agcgtaatcc ggcctggcgg 60
   cgtggagcag caaggccagc ggcaggagca aggcggtcac tggtaaggcc atggtgaatt 120
   ccccc 125
<210> 14
   <211> 8
   <212> PRT
   <213> Homo sapiens
<220>
   <221> MISC_FEATURE
   <223> CCK-8S
<220>
   <221> MOD_RES
   <222> (2).. (2)
   <223> SULFATATION
<400> 14

## Description

### TECHNICAL FIELD

The present invention uses a gene encoding a G-protein coupled receptor and the gene product thereof. More specifically, this invention uses gene encoding a G-protein coupled receptor for which cholecystokinin octapeptide sulfated form (hereunder; may also be referred to as "CCK-8S") acts as a ligand, and the gene product thereof. Further specifically, this invention uses a DNA encoding a protein having a function as a G-protein coupled receptor or the complementary strand thereof, a DNA comprising a partial base sequence of the DNA, a recombinant vector containing the above DNA or the complementary strand thereof, and a transformant having the recombinant vector introduced therein. The invention also uses a protein having a function as a G-protein coupled receptor, and an antibody against the protein. The invention relates to a method of identifying a compound that inhibits or promotes a function of the protein . The invention further relates to a method of identifying an agonist of the above protein. attributable to a decrease in CCK-8S and/or a decrease in the function thereof can be e.g. dementia (including Alzheimer's disease), anxiety disorders, obesity and diabetes. A disease associated with angiogenesis can be for example, cerebral infarction, cerebral contusion or tumor disease

### BACKGROUND ART

JP 2003/284573 discloses amino acid sequences which are similar to the amino acid sequence defined in SEQ ID NO: 2 of the present application.

JP 11/032766 and Shiratsuchi T. et al. (1997) Cytogen3et Cell Genet., vol. 79, p. 103-108 describe the cloning of the BAI2 receptor gene and splice variants thereof.

EP 1 270 724 A2 discloses methods to identify ligands, agonists and antagonists for human BAI2. EP 1 270 724 A2 also refers to a database entry having an accession number ADC86417 that discloses a nucleotide encoding human GPCR protein BAI2 (SEQ ID NO: 870) which is similar to SEQ ID NO: 1 of the present invention.

Kee Hae Jin et. al. (2002) Journal of Cerebral Blood Flow and Metabolism, vol. 22, no. 9, p. 1054-1067 discloses the amino acid sequence of human and murine BAI2 receptor and splice isoforms thereof.

Database entry having an accession number ABP81929 discloses an amino acid sequence of BAIP2 and nucleotide sequences coding therefore.

The G-protein coupled receptor (hereunder, also referred to as "GPCR") is a receptor for light, odor and flavor and, at the same time, is also a hormone and neurotransmitter receptor and serves as an important sensor of cells in living organisms ranging from yeasts to humans.

GPCR is a glycoprotein that is present in cell membrane that is one kind of seven-span transmembrane receptor that has the structural characteristic of having seven cell membrane spanning domains, and it constitutes a super family with a many members. One thousand or more GPCR genes have already been identified, and studies are proceeding in relation to the three dimensional structure of GPCR, extracellular stimulators (hereunder called "ligand", where the term "ligand" refers to a substance having the ability to bind with a receptor) for GPCR, intracellular signal transduction pathways through GPCR, and the functions thereof and the like.

When GPCR receives stimulation from a ligand it binds with G protein that is present inside the cell. G protein is a protein that couples with GPCR and has a function as a transduction factor for signals into the cell. G protein is broadly classified into a several kinds of families based on functions with respect to various factors (hereunder, referred to as "effector") involved in signal transduction in the intracellular signal transduction pathways and difference in the genes encoding the protein. The G proteins that belong to each family are trimers comprising three subunits called α, β and γ, and normally guanosine 5'-diphosphate (GDP) is bound specifically to α-subunit. GDP-bound G protein is an inactive form that does not exhibit an action with respect to an effector. When GPCR is stimulated by a ligand, an exchange reaction occurs between GDP binding to G protein and guanosine 5'-triphosphate (GTP) present in the cell, whereby the GDP is released from G protein and G protein then binds to GTP to form GTP-bound G protein. GTP-bound G protein is referred to as an active form, and it rapidly dissociates into α-subunit bound with GTP (αGTP) and a dimer (βγ) comprising β- and γ- subunits. αGTP and βγ directly act on an effector (for example, a calcium ion channel or a potassium ion channel) to activate the intracellular signal transduction pathway, and as a result, induce various physiological responses.

Amongst the GPCR superfamily, human brain angiogenesis inhibitor 2 (hereunder, abbreviated as hBAI2) is classified into class B (secretin like) and its gene is registered in GenBank under accession number AB 005298.

Although a report (Non-Patent Literature 1) exists relating to the sequence information and expression distribution of hBAI2, neither the function of hBAI2 nor its involvement in disease has been reported. However, based on a structural comparison with BAI1, a homolog of BAI2, it is considered that thrombospondin type I domain (hereunder referred to as "TSP-I domain") is present in the extracellular domain (Non-Patent Literature 2). TSP-I domain is a characteristic domain recognized in a region comprising the amino acid sequence from position 385 to position 522 in thrombospondin, and it is known to be involved in the extracellular matrix of thrombospondin and as an important function domain for angiogenesis inhibiting ability.

Regarding hBAI1, it has been reported that its expression is specifically high in human brain, that a domain having angiogenesis inhibitory ability is present in the extracellular region, that there is a possibility that it is subject to expression control by p53 and the like, suggesting the possibility that this gene is involved in some way in a mechanism relating to angiogenesis in the brain (Non-Patent Literature 1-4).

Regarding mouse BAI2 that is a gene associated with hBAI2, it is reported that a negative correlation is observed in the expression amount between BAI2 and vascular endothelial growth factor in brain tissue of cerebral ischemia model rat, and it is considered that, similarly to hBAI1, mouse BAI2 may be involved in angiogenesis. Further, the existence of a splice variant thereof (RNA species produced by splicing of mRNA) is also reported (Non-Patent Literature 3).

Even though it is predicted from the sequence information that both hBAI1 and hBAI2 belong to the GPCR family, no report can be found that mentions their functions as GPCR, including information regarding a ligand.

Meanwhile, cholecystokinin (hereunder, abbreviated as "CCK") is known as a gastrointestinal hormone released from endocrine cell in duodenal mucous membrane. CCK is secreted accompanying intake of fat, and promotes gallbladder contraction and pancreatic enzyme secretion. It exhibits actions in the digestive organs including gallbladder contraction, promotion of pancreatic enzyme secretion and stimulation of intestinal movement. CCK is also considered a signaling substance that imparts a sensation of satiety to cerebral neurons.

CCK is a straight-chain polypeptide consisting of 33 amino acid residues (hereunder, referred to as "CCK-33"), and it is known that the seventh tyrosine residue from the C-terminal side is sulfated. By post-translational processing of CCK, fragments of several lengths that have different cleavage sites on the N-terminal side, such as CCK-4, CCK-8, CCK-12, CCK-33 and CCK-58, are produced. It has been verified that the physiological activity and amount of each of these fragments are different. Further, cerulein that is extracted from the skin of frog has been reported as a compound that has a similar chemical structure to CCK and which exhibits the same biological activity.

CCK is widely distributed in the brain and, for example, it has been observed in large amounts in frontal cortex, hippocampus, amygdaloid body, and hypothalamus, and its action in the central nerves, such as involvement in analgesia, sedation, food intake control, memory and learning is also reported. CCK is partially colocalized with DA (Dopamine) and GABA (γ-aminobutyric acid), and its interaction with 5HT (serotonin; 5-hydroxytryptamine)-functioned nervous system and the like has also been reported. It has also been reported that release of CCK is adjusted by GABA. CCK-8 and CCK-4 have mainly been reported as CCK exhibiting bioactivity that is present in the brain. CCK-8 that is present in the brain is a sulfated type (CCK-8S) in which the seventh tyrosine residue from the C-terminal side is sulfated.

Recently, it has been reported that CCK is essential for memory retention. For example, it has been clarified that absence of CCK-8S makes it difficult to recall memory to conscious level and translate it into action, and that CCK-4 (a C-terminus tetrapeptide of CCK-33) obstructs mnemonic retrieval.

CCK-A receptor and CCK-B receptor have been reported as CCK receptors. These are both G-protein coupled receptors. Expression of CCK-A receptor is detected in tissues and cells originating in the alimentary canal, and in leukocytes and the like in the blood. CCK-A receptor is involved in alimentary regulation in the intracellular signal transduction pathway, for example, through promotion of effectors such as phospholipase C and adenyl cyclase. Meanwhile, expression of CCK-B receptor is detected in tissues and cells originating in the brain and alimentary canal, and in leukocytes and the like in the blood. CCK-B receptor is also referred to as "gastrin receptor", and is involved in alimentary regulation and cell proliferation in the intracellular signal transduction pathway, for example, through promotion of effectors such as phospholipase C and intracellular calcium ion influx. In recent years, attention is being focused on the relation between CCK-B receptor and anxiety.

Non-Patent Literature 1: Shiratsuchi, T. et al., "Cytogenetics and cell genetics", 1997, Vol. 79, p. 103-108.
Non-Patent Literature 2: Nishimori, H. et al., "Oncogene", 1997, Vol. 15, p. 2145-2150.
Non-Patent Literature 3: Kee, H. J. et al., "Journal of Cerebral Blood Flow and Metabolism", 2002, Vol. 22, p. 1054-1067.
Non-Patent Literature 4: Kaur, B. et al., "American Journal of Pathology", 2003, Vol. 162; p. 19-27.

### DISCLOSURE OF THE INVENTION

### [Problems to be Solved by the Invention]

GPCR serves as an important sensor of cells *in vivo*, and is a leading target molecule in the developing remedies for various diseases. Although a large number of GPCRs have already been found, identification of a novel GPCR can be expected to make a large contribution in the field of pharmaceutical development.
Another object of the present invention is to find and provide a ligand of a protein having a function that is equivalent to GPCR. A method of producing the protein, a recombinant vector containing the gene and a transformant having the recombinant vector introduced therein are described. Also described is an antibody against the protein. An object of the present invention is to provide a means for identifying a compound that inhibits or promotes the function of the protein.

### [Means for Solving the Problems]

The present inventors conducted concentrated studies and found a protein, which is considered to be a GPCR, originating in a functional membrane protein receptor that has a seven-span transmembrane domain as well as a gene encoding the protein, and succeeded in acquiring the gene product thereof using the gene. The present inventors clarified that the protein expressed on cell membrane in animal cells that expressed the gene, and that a physiological response was produced by ligand stimulation through intracellular signal transduction. It was also clarified that the protein interacted with a protein involved in intracellular signal transduction in the C-terminal region thereof, and also that it had three TSP-I domains that were known to be associated with an angiogenesis inhibiting function in its amino acid sequence. Further, it was found that CCK-8S acted as a ligand of the functional membrane protein receptor.

Thus, the present invention uses a DNA selected from the following group:
(i) a DNA consisting of a base sequence represented by SEQ ID NO: 1 of the sequence listing or a complementary strand thereof;
(ii) a DNA containing the DNA of (i) or a complementary strand thereof;
(iii) a DNA having homology of at least 70% with the base sequence of the DNA of (i) or (ii) and encoding a protein having an equivalent function to a G-protein coupled receptor, or a complementary strand thereof;
(iv) a DNA comprising a base sequence having a variation including a deletion, a substitution and an addition of one to several nucleotides in the base sequence of the DNA according to any one of (i) to (ii) and encoding a protein having an equivalent function to a G-protein coupled receptor, or a complementary strand thereof; and
(v) a DNA hybridizing under stringent conditions with the DNA according to any one of (i) to (iv) and encoding a protein having an equivalent function to a G-protein coupled receptor, or a complementary strand thereof.

The present invention also uses a DNA consisting of a partial base sequence of the aforementioned DNA.

The present invention further uses the aforementioned DNA, wherein the equivalent function to a G-protein coupled receptor is a function that activates a G protein by binding with a ligand.

The present invention still further uses the aforementioned DNA, wherein the ligand is a peptide selected from the following group:
(i) cholecystokinin octapeptide sulfated form (SEQ ID NO: 14, hereunder abbreviated as "CCK-8S"); and
(ii) a peptide comprising an amino acid sequence having a substitution of one homologous amino acid in the amino acid sequence of CCK-8S except for a sulfated tyrosine residue at the seventh position from the C-terminus of the peptide, and a function that induces an increase of an intracellular calcium concentration or a change in a cell membrane potential in a cell expressing the DNA usable in the present invention.

The present invention also uses a recombinant vector containing the aforementioned DNA.

The present invention further uses the aforementioned recombinant vector, wherein the recombinant vector is a recombinant expression vector.

The present invention still further uses a transformant that was introduced with the aforementioned recombinant vector.

The present invention also uses a transformant derived from an animal cell that was introduced with the aforementioned recombinant vector.

The present invention further uses a cell line Deposit No. FERM BP-10101.

The present invention still further uses protein encoded by the aforementioned DNA.

The present invention also uses a protein selected from the following group:
(i) a protein consisting of an amino acid sequence represented by SEQ ID NO: 2 of the sequence listing;
(ii) a protein containing the protein of (i);
(iii) a protein having homology of at least 70% with the amino acid sequence of the protein of (i) or (ii) and having an equivalent function to a G-protein coupled receptor; and
(iv) a protein comprising an amino acid sequence having a variation including a deletion, a substitution and an addition of one to several amino acids in the amino acid sequence of the protein according to any one of (i) to (iii), and having an equivalent function to a G-protein coupled receptor.

The present invention further uses a protein consisting of a partial sequence of an amino acid sequence represented by SEQ ID NO: 2 of the sequence listing.

The present invention still further uses the aforementioned protein, which has an equivalent function to a G-protein coupled receptor.

The present invention also uses the aforementioned protein, which has an angiogenesis inhibiting function.

The present invention further uses the aforementioned protein, which has a function that interacts with a protein having guanylate kinase activity and/or a protein having an intercellular adhesion function.

The present invention still further uses a protein selected from the following group, which is a protein having an equivalent function to a G-protein coupled receptor, a function that interacts with a protein having an intercellular adhesion function and/or a protein having guanylate kinase activity, and an angiogenesis inhibiting function:
(i) a protein consisting of an amino acid sequence represented by SEQ ID NO: 2 of the sequence listing;
(ii) a protein containing the protein of (i);
(iii) a protein having homology of at least 70% with the amino acid sequence of the protein of (i) or (ii); and
(iv) a protein comprising an amino acid sequence having a variation including a deletion, a substitution or an addition of one to several amino acids in the amino acid sequence of the protein according to any one of (i) to (iii).

The present invention also uses the aforementioned protein, wherein the equivalent function to a G-protein coupled receptor is a function that activates a G protein by binding with a ligand.

The present invention further uses the aforementioned protein, which has a function that generates a change in a cell membrane potential when a ligand is bound to the protein on a cell.

The present invention still further uses the aforementioned protein, which has a function that increases an intracellular calcium concentration when a ligand is bound to the protein on a cell.

The present invention also uses the aforementioned protein, wherein the ligand is a peptide selected from the following group:
(i) cholecystokinin octapeptide sulfated form (SEQ ID NO: 14, hereunder abbreviated as "CCK-8S");
(ii) a peptide comprising an amino acid sequence having a substitution of one homologous amino acid in the amino acid sequence of CCK-8S except for a sulfated tyrosine residue at the seventh position from the C-terminus of the peptide, and having a function that induces an increase of an intracellular calcium concentration or a change in a cell membrane potential in a cell expressing the DNA usable in the present invention.

The specification describes a method for producing the aforementioned protein, comprising culturing the aforementioned transformant.

The present invention uses an antibody that immunologically recognizes the aforementioned protein. contacted with the protein.

The present invention still further relates to the aforementioned identification method, selecting a test compound or a test substance that changes a cell membrane potential is selecting a test substance that generates a current variation that is distinctive of a G-protein coupled receptor.

The present invention relates to a method of identifying a compound that inhibits or promotes a binding of the aforementioned protein with a ligand or a function of the protein, comprising using at least one member of the group consisting of the aforementioned DNA, the aforementioned recombinant vector, the aforementioned transformant, the aforementioned cell line, the aforementioned protein, and the aforementioned antibody. The present invention still further relates to methods as claimed.

The present invention still further relates to a method of identifying a compound that inhibits or promotes a function of the aforementioned protein, comprising allowing the aforementioned transformant and a test compound to coexist under a condition that enables interaction with the transformant or the aforementioned cell line, introducing a system that measures a function of a protein that is expressed on a cell membrane of the transformant, and determining whether or not the test compound inhibits or promotes the function of the protein by detecting the existence or non-existence of, or a change in, the function of the protein.

The present invention also relates to the aforementioned identification method, wherein the system that measures a function of a protein that is expressed on a cell membrane of the transformant is a system that measures a change in an intracellular calcium concentration produced by addition of a ligand, and the detection of the existence or non-existence of, or a change in, a function of the protein is detection of a change in an intracellular calcium concentration.

The present invention further relates to the aforementioned identification method, wherein the system that measures a function of a protein that is expressed on a cell membrane of the transformant is a system that measures a change in a membrane potential produced by addition of a ligand, and the detection of the existence or non-existence of, or a change in, a function of the protein is detection of a change in a membrane potential.

The present invention still further relates to the aforementioned identification method, wherein the ligand is a peptide selected from the following group:
(i) cholecystokinin octapeptide sulfated form (SEQ ID NO: 14, hereunder abbreviated as "CCK-8S"); and
(ii) a peptide comprising an amino acid sequence having a substitution of one homologous amino acid in the amino acid sequence of CCK-8S except for a sulfated tyrosine residue at the seventh position from the C-terminus of the peptide; and having a function that induces an increase of an intracellular calcium concentration or a change in a cell membrane potential in a cell expressing the DNA usable in the present invention.

The present invention still further relates to the aforementioned assay method, comprising using at least one member of the group consisting of the aforementioned DNA, the aforementioned recombinant vector, the aforementioned transformant, the aforementioned cell line, the aforementioned protein, and the aforementioned antibody.

### [Advantages of the Invention]

A functional membrane protein receptor-derived protein that has a seven-span transmembrane domain which is considered to be a GPCR and a DNA encoding the protein are described. Since this protein has three TSP-I domains, it is thought to be involved in angiogenesis inhibition. Further, since the protein interacts with a MAGUK (membrane-associated guanylate kinase homolog) family protein (protein having guanylate kinase activity and an intercellular adhesion function) in the C-terminal region thereof, the protein may be involved in the intercellular adhesion function of cells.

According to the present invention, it was also found that CCK-8S acted as a ligand of the functional membrane protein receptor. Since CCK-8S induced a biological response through the functional membrane protein receptor of the present invention at the low concentration of 1 nM, it is considered that CCK-8S is an *in vivo* ligand of the functional membrane protein receptor. It is reported that CCK-8S is essential for memory retention, for example, that absence of CCK-8S makes it difficult to recall memory to the conscious level and translate it into action. It is considered that the action of CCK-8S with respect to this kind of neurologic function occurs through the functional membrane protein receptor described in the present invention. It is therefore considered that an agonist of the functional membrane protein receptor described in the present invention has an action that is equivalent to that of CCK-8S, i.e. an action that modifies a neurologic function such as memory and the like.

According to the present invention, it is possible to identify and acquire an agonist of a functional membrane protein receptor on which CCK-8S acts. Also described is the possibility to modify the neurologic function using the agonist. For example, it is possible to retain memory using the agonist. Further, it is possible to alleviate a disease or symptoms accompanying hindrance of neurologic function using the agonist.

It is possible to diagnose, prevent and/or treat a disease caused by an abnormality in the protein and/or DNA, for example, a disease associated with angiogenesis. Specific examples of this type of disease include cerebral infarction, cerebral contusion and tumor disease. Further, since the protein is a functional membrane protein receptor, it is also possible to diagnose, prevent and/or treat a disease caused by a decline or disappearance or the like in the function and/or amount of a ligand thereof, for example, CCK-8S. Examples of this kind of disease include a disease accompanying damage to the neurologic function of memory or the like, and specific examples thereof include Alzheimer's disease.

### BRIEF DESCRIPTION OF THE DRAWINGS

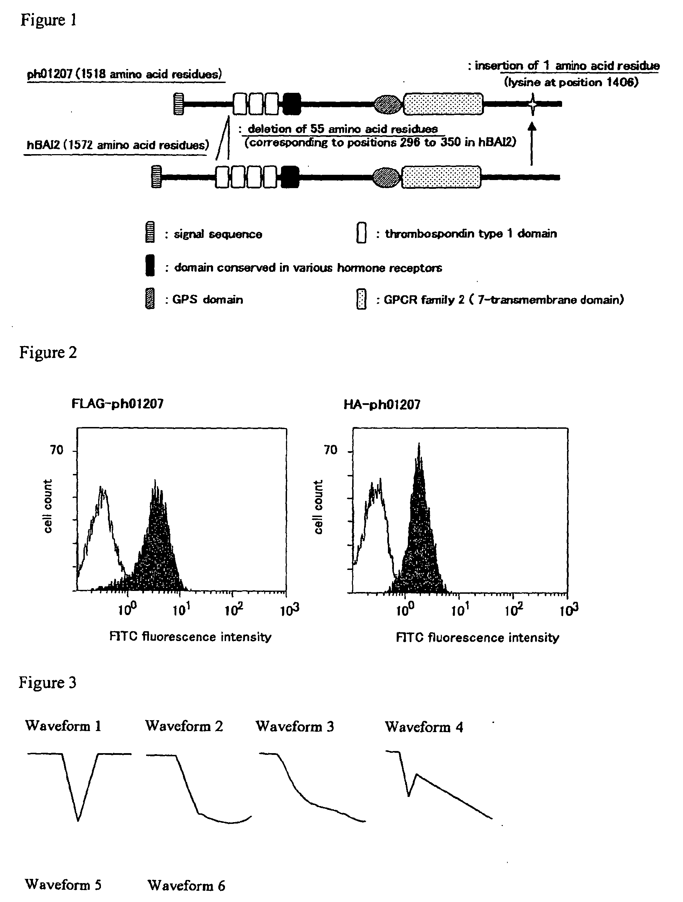

Figure 1 is a schematic diagram illustrating a comparison between a function domain of a peptide comprising the amino acid sequence represented by SEQ ID NO: 2 and a peptide encoded by hBAI2. (Example 1)
Figure 2 is a view illustrating that, when cDNA clone ph01207 was expressed in CHO-K1 cells, an animal cell line, as a FLAG-tag fusion protein and a HA-tag fusion protein, respectively, these proteins expressed on the cell membrane (the left panel and right panel, respectively). Detection of the protein on the cell membrane was analyzed by fluorocytometry using anti-FLAG-tag antibody and anti-HA-tag antibody. In the figure, the region shown in black indicates cells that expressed each tag fusion protein and the region shown in white indicates control cells that did not express the proteins. (Example 2)
Figure 3 is a view illustrating waveforms that can be observed when the ligand response of GPCR is measured by variations in membrane potential of cells (waveform produced by GPCR-specific response (waveform 1), waveforms produced by artificial elements and the like (waveform 2-4), and waveforms recognized at the time of no response (waveforms 5 and 6)). (Example 3)

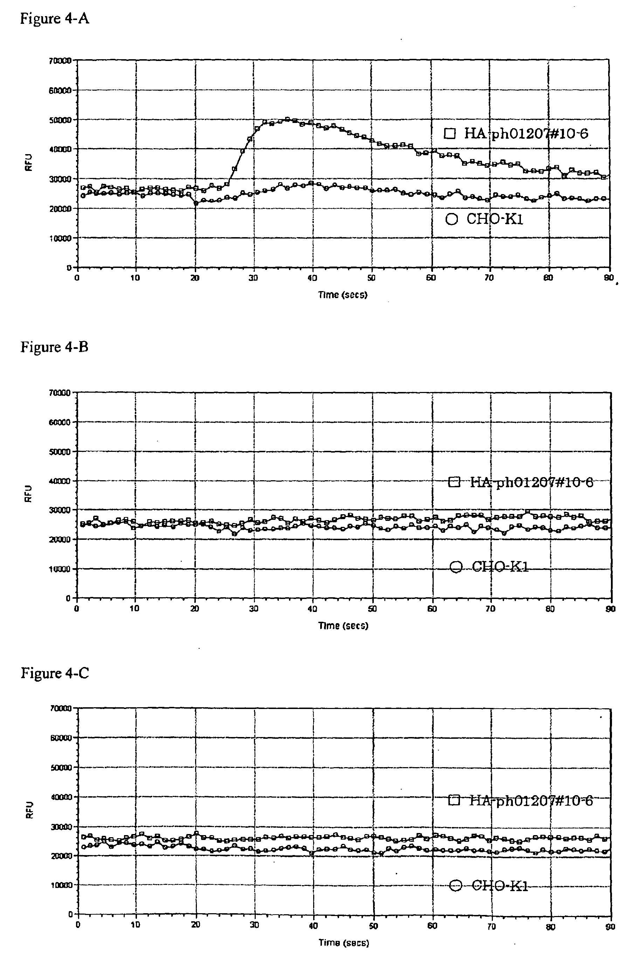

Figure 4-A is a view illustrating that intracellular Ca²⁺ concentration increased with addition of 1 nM CCK-8S (SEQ ID NO: 14) in CHO-K1 cell line (HA-ph01207#10-6) stably expressing cDNA clone ph01207 as a HA-tag fusion protein. In contrast, in CHO-K1 cell line that was not transfected with this cDNA, an increase in intracellular Ca²⁺ concentration with addition of CCK-8S (SEQ ID NO: 14) was not observed. The horizontal axis shows the time from addition of CCK-8S (SEQ ID NO: 14) to cells, and the longitudinal axis shows intracellular Ca²⁺ concentration. (Example 5)
Figure 4-B is a view illustrating that an increase in intracellular Ca²⁺ concentration with addition of 1 nM CCK-8NS was not observed in CHO-K1 cell line (HA-ph01207#10-6) stably expressing cDNA clone ph01207 as a HA-tag fusion protein. Although CCK-8NS (CCK-8 Nonsulfated form) is a CCK octapeptide consisting of the same amino acid sequence as CCK-8S, it is a peptide in which the seventh tyrosine residue from the C-terminus is not sulfated. An increase in intracellular Ca²⁺ concentration with addition of CCK-8NS was also not observed in a CHO-K1 cell line that was not transfected with cDNA clone ph01207. The horizontal axis shows the time from addition of CCK-8NS to cells, and the longitudinal axis shows intracellular Ca²⁺ concentration. (Example 5)
Figure 4-C is a view illustrating that an increase in intracellular Ca²⁺ concentration with addition of 1 nM CCK-4 was not observed in CHO-K1 cell line (HA-ph01207#10-6) stably expressing cDNA clone ph01207 as a HA-tag fusion protein. An increase in intracellular Ca²⁺ concentration with addition of CCK-4 was also not observed in CHO-K1 cell line that was not transfected with the cDNA. The horizontal axis shows the time from addition of CCK-4 to cells, and the longitudinal axis shows intracellular Ca²⁺ concentration. (Example 5)

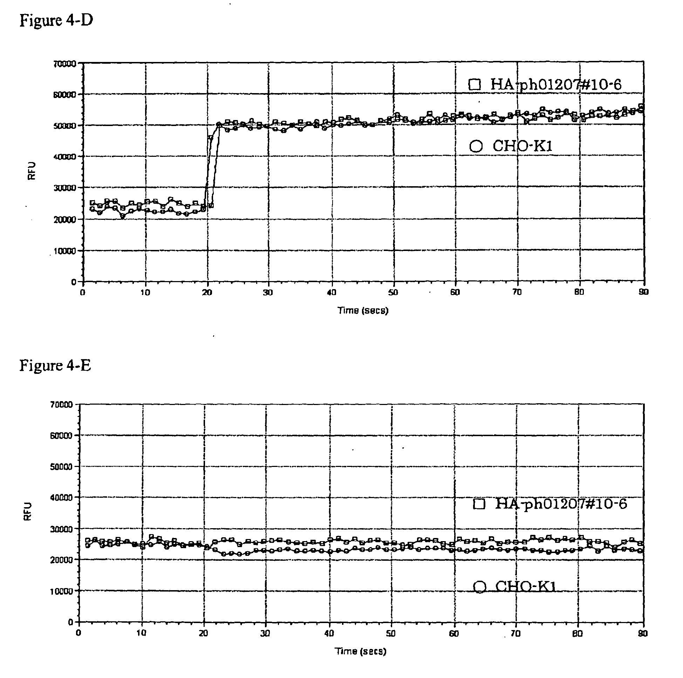

Figure 4-D is a view illustrating that intracellular Ca²⁺ concentration increased with addition of 10 µM calcium ionophore A23187 in CHO-K1 cell line (HA-ph01207# 10-6) stably expressing cDNA clone ph01207 as a HA-tag fusion protein. An increase in intracellular Ca²⁺ concentration with addition of A23187 was also observed in CHO-K1 cell line that was not transfected with the cDNA. The horizontal axis shows the time from addition of A23187 to cells, and the longitudinal axis shows intracellular Ca²⁺ concentration. (Example 5)
Figure 4-E is a view illustrating intracellular Ca²⁺ concentrations after addition of a buffer in CHO-K1 cell line (HA-ph01207#10-6) stably expressing cDNA clone ph01207 as a HA-tag fusion protein and CHO-K1 cell line that was not transfected with the cDNA. The horizontal axis shows the time from addition of the buffer to cells, and the longitudinal axis shows intracellular Ca²⁺ concentration. (Example 5)

### DETAILED DESCRIPTION

The present invention claims the benefit of priority from Japanese Patent Application Nos. 2003-389624, 2004-130371 and 2004-304780.

Examples relating to the present invention are described in further detail hereunder. However, it will be understood that the following detailed description is exemplary and for the purpose of explanation only, and is not intended to limit the present invention in any way.

In the present specification, the term "protein" is sometimes used as a generic term that refers to an isolated or synthetic full length protein, an isolated or synthetic full length polypeptide, or an isolated or synthetic full length oligopeptide. In this case, a protein, polypeptide or oligopeptide has a minimum size of two amino acids. Hereunder, one character or three characters may be use when representing an amino acid.

The present invention describes a novel functional membrane protein receptor-derived protein having a seven-span transmembrane domain that is considered to be a GPCR, and a DNA encoding the protein. Since this protein has three TSP-I domains, it is considered to be involved in angiogenesis inhibition. Further, since the protein interacts with a MAGUK family protein (a protein having guanylate kinase activity and an intercellular adhesion function) at the C-terminal region thereof, there is a possibility that the protein is associated with an intercellular adhesion function of cells.

It was also found by the present invention that CCK-8S (SEQ ID NO: 14) acts as a ligand of a functional membrane protein receptor of the present protein. Since CCK-8S (SEQ ID NO: 14) initiated a biological response through the functional membrane protein receptor at the low concentration of 1 nM, the inventors considered that CCK-8S (SEQ ID NO: 14) is an *in-vivo* ligand of the functional membrane protein receptor. It has been reported that CCK-8S is essential for memory retention, for example, that absence of CCK-8S (SEQ ID NO: 14) makes it difficult to recall memory to a conscious level and translate it into action. The inventors considered that the action of CCK-8S (SEQ ID NO: 14) with respect to this kind of neurologic function takes place through the functional membrane protein receptor of the present invention.

### (DNA)

The DNA used in the invention is a cDNA clone having a region encoding a seven-span transmembrane domain that was identified from a long-chain cDNA library derived from the human brain. The long-chain cDNA library derived from the human brain is a cDNA library comprising cDNA clones whose complete base sequence was determined after isolating cDNA fragments by dbEST (database of Expressed Sequence Tags) analysis from a cDNA library constructed by an ordinary method employing commercially available polyA⁺RNA (Clontech Inc.; catalog number 6516-1, 6525-1 and 6578-1) derived from the human brain, fetal brain and cerebral hippocampus as a starting material.

As examples of a specific form of the DNA used in the present invention, a DNA. comprising the base sequence represented by SEQ ID NO: 1 of the sequence listing or the complementary sequence thereof may be mentioned. In this specification, a DNA comprising the complementary sequence of a certain DNA may also be referred to as a "complementary strand". The DNA comprising the base sequence represented by SEQ ID NO: 1 comprises a novel base sequence of 4557 bps that includes an open reading frame (ORF) encoding 1518 amino acid residues (SEQ ID NO: 2) having a segment that is predicted to be a signal sequence (20 amino acid residues from N terminus).

It was clarified by homology search that the DNA comprising the base sequence represented by SEQ ID NO: 1 has homology with hBAI2 (GenBank, accession number AB005298). It is considered that, in addition to four TSP-I domains, the protein encoded by hBAI2 has a GPS domain and a GPCR family 2 domain (seven-span transmembrane domain).

It was found that the structural characteristics of the protein (SEQ ID NO: 2) that is encoded by the DNA comprising the base sequence represented by SEQ ID NO: 1 include, in addition to three TSP-I domains, the presence of a GPS domain and a GPCR family 2 domain (see Figure 1).

It was clarified by sequence comparison that there is a difference in the number of TSP-I domains and the C-terminal amino acid sequence between the protein (SEQ ID NO: 2) encoded by the DNA comprising the base sequence represented by SEQ ID NO: 1 and the protein encoded by hBAI2 (see Figure 1). More specifically, it was found that 55 amino acid residues including one TSP-I domain on the N-terminal region side of the amino acid sequence of the protein encoded by hBAI2 are deleted in the present protein (SEQ ID NO: 2), and that in the C-terminal region, one amino acid residue that corresponds to lysine at position 1406 is inserted in the present protein (SEQ ID NO: 2). There is thus a possibility that the present protein (SEQ ID NO: 2) is a splicing variant ofhBAI2.

The DNA comprising the base sequence represented by SEQ ID NO: 1 was expressed on the cell membrane when expressed in an animal cell. Further, in the animal cell that expressed the DNA, a response to the action of a ligand was obtained. It was also clarified that the gene product of the present DNA can interact with MAGUK family proteins DLG2, DLG3 and DLG4 or AIP1, MAGI3 and the like in the C-terminal region thereof. MAGUK family proteins have a PDZ domain that recognizes the last C-terminal amino acid sequence of a target protein in protein interaction, and it is considered that by localizing on the cell membrane to interact with a membrane protein such as a receptor or an ion channel, the proteins participate in signal transduction from these membrane proteins to contribute to intercellular adhesion and the like. Interaction between hBAI2 and MAGUK family proteins has similarly been recognized. Meanwhile, it has been reported that hBAI1, an hBAI2 homologue, binds with BAP1 (BAI1-associated protein 1), one of the MAGUK family proteins, through a partial sequence (QTEV: SEQ ID NO: 3) of the distal region of hBAI1 (Shiratsuchi, T. et al., "Biochemical and Biophysical Research Communications", 1998, Vol. 247, p.597-604). This sequence (QTEV: SEQ ID NO: 3) is preserved in the C-terminal region of both the protein encoded by the DNA comprising the base sequence represented by SEQ ID NO: 1 and the protein encoded by hBAI2, and there is therefore a possibility that the proteins interact with a protein having a PDZ domain in this sequence segment.

As used herein, the term "interaction" refers to, for example, two homologous or distinct proteins specifically acting together, and the function of one or both of the proteins changing as a result, for example, enhancing or decreasing. The phrase "specifically acting" refers to acting more selectively with the protein involved in the action than with other proteins. The term interaction, for example, includes binding of two distinct proteins or activation of one protein being caused by the other protein.

It was clarified on the basis of the structural characteristics and functional characteristics of the gene product of the DNA comprising the base sequence represented by SEQ ID NO: 1 that the gene product is a functional membrane protein receptor. Further, because it has a seven-span transmembrane domain, it was considered that it is one of the GPCRs having a function that binds to G protein by ligand stimulation to activate the G protein, promote intracellular signal transduction, and induce a cellular biological response.

Further, since it has TSP-I domains, it was predicted that the protein encoded by the DNA comprising the base sequence represented by SEQ ID NO: 1 has an angiogenesis inhibiting function.

Another form of the DNA used in the present invention is a DNA containing the base sequence represented by SEQ ID NO: 1 or the complementary base sequence of that base sequence.

A DNA having sequence homology with the DNA used in the present invention and having a similarity relating to structural characteristics or the biological function of the protein encoded by the DNA is also described

A suitable sequence homology is normally 50% or more homology with the entire base sequence, and preferably is at least 70%. A suitable sequence homology is more preferably greater than 70%, further preferably is 80% or more, and still further preferably is 90% or more.

Examples of the structural characteristics include a seven-span transmembrane domain coding region and a TSP-I domain coding region. A preferable DNA has a sequence homology in these types of regions that is preferably at least 70%, more preferably greater than 70%, further preferably is 80% or more, and still further preferably is 90% or more. It is further preferable that these domains are domains that retain a function thereof, for example, a function that localizes a protein including the domain on a membrane or an angiogenesis inhibiting function.

As an example of one of the biological functions of the protein encode by the DNA used in the present invention, a function as a membrane protein receptor can be mentioned. The phrase "function as a membrane protein receptor" refers to a function that promotes intracellular signal transduction through the action of a ligand to induce a biological response in an animal cell by expression as a membrane protein when expressed in the cell. For example, an equivalent function to a GPCR may be mentioned. The phrase "equivalent function to a GPCR" refers to a function which binds to G protein by action of a ligand to activate the G protein, and promotes intracellular signal transduction to induce a biological response in the cell. As described later, CCK-8S (SEQ ID NO: 14) was found as one of the ligands of the membrane protein receptor encoded by that present DNA. Accordingly, the DNA used in the present invention preferably has sequence homology with the DNA comprising the base sequence represented by SEQ ID NO: 1 and encodes a protein that exhibits an equivalent function to a GPCR through the action of CCK-8S (SEQ ID NO: 14).

As specific examples of a biological response of a cell, a change in cell membrane potential or a change in intracellular calcium concentration can be mentioned. A change in cell membrane potential or a change in intracellular calcium concentration can be measured by a known method. A change in cell membrane potential can be detected, for example, by expressing the DNA of the present invention in *Xenopus laevis* oocyte, measuring the amount of current generated specifically for membrane protein receptor in the presence and absence of ligand stimulation, and comparing the amounts of current. A change in intracellular calcium concentration can be detected, for example, by incorporating into the cell a fluorescent substance that can bind with calcium ion, eliciting a fluorescence phenomenon by excitation light in the presence and absence of ligand stimulation, and comparing the fluorescence amounts. Examples of a ligand include a sample prepared from a cell or biotissue in which expression of the present DNA was recognized. Sample preparation can be carried out, for example, by culturing cells or tissue according to a known method, and then employing a method which obtains the culture supernatant by centrifuging or the like, or a method which disrupts or lyses the cells or tissue by a known method. A ligand can also be acquired for use by purification from these samples by a known protein purification method, for example, gel filtration chromatography. Specific examples of a ligand include, but are not limited to, culture supernatant of the HeLa cell line that was used in the present example, and any substance can be used as a ligand as long as it can act on the gene product of the present DNA that expressed in a cell to induce a biological response in the cell. More preferably, CCK-8S (SEQ ID NO: 14) can be exemplified as a ligand.

As an example of a different biological function, a function that interacts with a protein having guanylate kinase activity and/or a cell adhesion function, for example a MAGUK family protein, may be mentioned. Specific examples of MAGUK family protein include DLG2, DLG3 DLG4, AIP1 and MAGI3.

The DNA used in the present invention includes a DNA comprising a base sequence having a variation including a deletion, substitution, addition or insertion of one or more, for example 1 to 100, preferably 1 to 30, more preferably 1 to 20, further preferably 1 to 10, and particularly preferably 1 to several nucleotides in the base sequence of the above DNA. A preferable DNA is a DNA of this kind that encodes a protein having the above described biological function. The degree of variation and the location thereof and the like are not particularly limited, as long as a DNA having the variation has similar structural characteristics as the above DNA and has a biological function that is equivalent to that of the protein encoded by the DNA comprising the base sequence represented by SEQ ID NO: 1. A DNA having this kind of variation may be a natural DNA or may be a DNA obtained by introduction of a variation on the basis of a gene existing in nature. Techniques for introducing a variation are known, for example, site-directed mutagenesis, genetic homologous recombination, primer extension, and polymerase chain reaction (hereunder, abbreviated as PCR), and these techniques can be used independently or in suitable combinations thereof. For example, a variation may be introduced in accordance with a method described in a book (Sambrook et al., Eds., "Molecular Cloning, A Laboratory Manual, 2nd Edition", 1989, Cold Spring Harbor Laboratory; Muramatsu S., Ed., "Labomanual Genetic Engineering", 1988, Maruzen Co., Ltd.) or by modifying these methods, and Ulmer's technique (Ulmer, K. M., "Science", 1983, Vol. 219, p.666-671) may also be utilized.

As a further example of the DNA used in the present invention, a DNA that hybridizes with the above described DNA under stringent conditions may also be mentioned. The hybridization conditions can, for example, be accordance with a method described in a book (Sambrook et al., Eds., "Molecular Cloning, A Laboratory Manual, 2nd Edition", 1989, Cold Spring Harbor Laboratory) or the like. More specifically, the phrase "under stringent conditions" refers to, for example, conditions of heating at 42 °C in a solution containing 6 × SSC, 0.5% SDS and 50% formamide, and then washing at 68 °C in a solution containing 0.1 × SSC and 0.5% SDS. As long as these DNAs are DNAs that hybridize with the present DNA, it is not necessary that they are DNAs having the complementary sequence thereof. Preferably, the DNA is a DNA encoding a protein having the above described biological function.

A still further form of the DNA used in according to the present invention may be a DNA fragment comprising a partial base sequence that is present in a designated region of the above described DNA. As a preferable example of this kind of region, a region encoding a fragment including a site at which a ligand acts that is a fragment of a protein encoded by the present DNA may be mentioned. A DNA fragment comprising a partial base sequence that is present in a region encoding a fragment including a site at which a ligand acts can be used in the production of a fragment including a site at which the ligand acts. A fragment including a site at which a ligand acts is useful for detecting an action of a ligand with respect to the present protein, for example, binding between the present protein and the ligand, or identifying a compound that promotes or inhibits the action. Alternatively, the fragment is useful for identifying a compound having the same action as a ligand with respect to the present protein, i.e. an agonist. The minimum unit of this kind of DNA fragment preferably comprises five or more consecutive nucleotides in the region, more preferably ten or more nucleotides, and further preferably 20 or more nucleotides. These DNA fragments can be prepared according to a known chemical synthesis method by designing a fragment having the target sequence in accordance with the base sequence information of the present DNA. As a simple and convenient method, the DNA fragments can be prepared using an automated DNA/RNA synthesizer. The present DNA fragments can be used as primers or probes for detecting the present DNA, or as primers for producing the present DNA. The primer preferably consists of 15 to 30 nucleotides, and more preferably 20 to 25 nucleotides. The probe preferably consists of 8 to 50 nucleotides, more preferably 17 to 35 nucleotides, and further preferably 17 to 30 nucleotides. If the length of a primer or probe is longer than a suitable length, the specificity will decrease due to an increase in false hybridization. Further, if the length is shorter than a suitable length, the specificity will decrease due to the occurrence of mismatches. When the present DNA fragment is a complementary DNA fragment to a sense strand encoding a protein, it can be used as an antisense oligonucleotide that inhibits expression of the present DNA. Since it is known that, generally, a DNA fragment consisting of approximately 20 nucleotides can inhibit Expression of a gene, the antisense oligonucleotide preferably consists of 15 or more nucleotides, and more preferably 20 or more nucleotides.

The DNA used in the present invention can be readily acquired by a known genetic engineering technique (refer to Sambrook et al., Eds., "Molecular Cloning, A Laboratory Manual, 2nd Edition", 1989, Cold Spring Harbor Laboratory; Muramatsu S., Ed., "Labomanual Genetic Engineering", 1988, Maruzen Co., Ltd.) based on sequence information regarding a specific example thereof disclosed according to the present invention, for example, the DNA comprising the base sequence represented by SEQ ID NO: 1 in the sequence listing.

More specifically, the DNA used in the present invention can be acquired by preparing a cDNA library in accordance with an ordinary method from a suitable origin in which expression of the DNA of the present invention is confirmed, and then selecting a desired clone from the library using a suitable probe or primer that is specific to the DNA. Examples of the cDNA origin include various cells or tissue in which expression of the present DNA is confirmed, or cultured cells derived from these, for example, human brain cells or the like. Isolation of total RNA from these origins, isolation and purification of mRNA, acquisition of cDNA and the cloning thereof and the like can each be carried out in accordance with an ordinary method. It is also possible to use a cDNA library that was constructed from commercially available polyA⁺RNA derived from the human brain, fetal brain or cerebral hippocampus. A method for selecting a desired clone from a cDNA library is also not particularly limited, and a commonly used method can be used. Examples thereof include a plaque hybridization method or colony hybridization method that uses a probe which binding selectively to the target DNA sequence, or a combination of these methods. As a probe used in this case, although a DNA or the like that was chemically synthesized on the basis of information relating to the base sequence of the present DNA can generally be used, the present DNA or a fragment thereof that was previously acquired can be preferably used. A sense primer and antisense primer that were designed on the basis of the base sequence information of the present DNA can also be used as this kind of probe.

Selection of a target clone from a cDNA library can be carried out, for example, by confirming an expression protein for each clone utilizing a known protein expression system, using the biological function thereof as an indicator.

In addition, a DNA/RNA amplification method according to PCR (Ulmer, K. M., "Science", 1983, Vol. 219, pp.666-671; Ehrlich, H. A., Ed., "PCR Technology. Principles and Applications for DNA Amplification", 1989, Stockton Press; Saiki, R. K., et al., "Science", 1985, Vol. 230, pp.1350-1354) can be favorably utilized to acquire the DNA of the present invention. When it is difficult to acquire full length cDNA from a cDNA library, a RACE method ("Jikken Igaku (Experimental Medicine)", 1994, VOl. 12, No. 6, p. 35-), and particularly the 5'-RACE method (Frohman, M. A., "Proceedings of The National Academy of Sciences of The United States of America", 1988, Vol. 85, No. 23, pp. 8998-9002) or the like can be favorably employed. Primers to be used for PCR can be suitably designed based on the base sequence information of the DNA, and obtained by synthesis in accordance with an ordinary method. Isolation and purification of amplified DNA/RNA fragments can be carried out according to an ordinary method. For example, isolation and purification of amplified DNA/RNA fragments can be carried out by gel electrophoresis or the like.

Determination of the base sequence of DNA obtained in this manner can be carried out by an ordinary method, for example, the dideoxy chain termination method ("Proceedings of The National Academy of Sciences of The United States of America", 1977, Vol. 74, pp.5463-54.67) or the Maxam-Gilbert method (Methods in Enzymology", 1980, Vol. 65, p.499-), or simply and conveniently using a commercially available sequencing kit or the like.

Although the DNA used in the present invention originates in human, DNA of a mammal that encodes a protein having biological functions that are equivalent to a protein encoded by the present DNA and also has structural homology with the present DNA, such as DNA of mouse, horse, sheep, cow, dog, monkey, cat, bear, rat or rabbit, is also included in the scope of the present invention.

The DNA used in the present invention may also be a DNA to the 5'-terminal side or 3'-terminal side of which one or more genes of, for example, enzymes such as glutathione S-transferase (GST), β-galactosidase, horseradish peroxidase (HRP) or alkaline phosphatase (ALP), or tag peptides such as His-tag, Myc-tag, HA-tag, FLAG-tag or Xpress-tag are attached, as long as the function thereof, for example, expression of the protein encoded by the DNA or the function of the expressed protein, is not inhibited. Attachment of these genes can be carried out by a commonly used genetic engineering technique, and is useful to facilitate detection of a gene or mRNA.

### (Vector)

Also used is a recombinant vector that contains the DNA used in the present invention. The recombinant vector can be obtained by inserting the present DNA into a suitable vector DNA.

The recombinant vector incorporating the DNA may be any kind of recombinant vector, as long as it is a recombinant vector into which the present DNA is incorporated. The vector DNA is not particularly limited as long as it can be replicated within a host, and it can be suitably selected in accordance with the kind of host and purpose of use. The vector DNA may be extracted from a substance existing in nature, or may be one in which one part of a DNA segment other than a segment necessary for replication has been deleted. As typical examples, vector DNA derived from a plasmid, a bacteriophage or a virus may be mentioned. Examples of plasmid DNA include a plasmid derived from *Escherichia coli,* a plasmid derived from *Bacillus subtilis,* and a plasmid derived from yeast. Examples of a bacteriophage DNA include λ phage. Examples of vector DNA derived from a virus include a vector derived from an animal virus such as retrovirus, vaccinia virus, adenovirus, papovavirus, SV 40, fowlpox virus, and pseudorabies virus, or a vector derived from an insect virus such as baculovirus. Other examples thereof include vector DNA derived from a transposon, an insertion element, or a yeast chromosome element. Alternatively, vector DNA obtained by combining two or more of these, for example, vector DNA (cosmid or phagemid or the like) produced by combining genetic elements of a plasmid and a bacteriophage may be employed. Further, an expression vector or cloning vector or the like can also be used in accordance with the object.

It is necessary for a gene to be incorporated into the vector so that the target function of the gene is exerted, and the vector should comprise at least the target gene sequence and a promoter. In addition to these elements, as desired, a genetic sequence that holds information relating to replication and control, for example, one or a plurality of genetic sequences combined according to a known method selected from the group consisting of a ribosome binding sequence, terminator, signal sequence, cis element such as an enhancer, splicing signal and selective marker can be incorporated into the vector DNA. As a selective marker, for example, dihydrofolate reductase gene, ampicillin-resistant gene and neomycin-resistant gene may be mentioned.

A known method can be applied to a method for incorporating the gene sequence of interest into the vector DNA. For example, a method can be used in which the gene sequence of interest is treated with a restriction enzyme to be cleaved at a specific site, subsequently mixed with similarly treated vector DNA, and finally reconnected using a ligase. Alternatively, a desired recombinant vector can also be obtained by ligating a suitable linker to the gene sequence of interest, and then inserting this into a multicloning site of a vector suited to the purpose.

### (Transformant)

Also used in the present invention is a transformant obtained by introducing vector DNA containing the DNA used in the present invention into a host. When using an expression vector as the vector DNA, the present DNA can be expressed, and a protein encoded by the DNA can also be produced. One or more vector DNA containing a desired gene other than the present DNA can also be introduced into the transformant.

Both prokaryotes and eukaryotes can be used as a host. Examples of the prokaryotes include bacteria belonging to *Escherichia,* such as *Escherichia coli,* bacteria belonging to *Bacillus,* such as *Bacillus subtilis,* bacteria belonging to *Pseudomonas,* such as *Pseudomonas putida,* and bacteria belonging to Rhizobium, such as *Rhizobium meliloti.* Examples of the eukaryotes include yeasts such as *Saccharomyces cerevisiae* and *Schizosaccharomyces pombe,* insect cells such as Sf9 and Sf21, and animal cells such as cells of monkey kidney (COS cells, Vero cells), Chinese hamster ovary cells (CHO cells), mouse L cells, rat GH3 cells, human FL cells or 293 EBNA cells, and *Xenopus laevis* oocyte. Preferably, animal cells are used.

Introduction of vector DNA into the host cell can be performed according to a known method, for example, by applying a standard method described in a book (Sambrook et al., Eds., "Molecular Cloning, A Laboratory Manual, 2nd Edition", 1989, Cold Spring Harbor Laboratory). Although a method which integrates the gene onto the chromosome may be mentioned as a more preferable method in consideration of the stability of the gene, an autonomous replication system that utilizes an extranuclear gene can be used as a simple method. As specific methods, calcium phosphate transfection, DEAE-dextran mediated transfection, microinjection, cationic lipid-mediated transfection, electroporation, transduction, scrape loading, ballistic introduction and infection may be mentioned.

When employing an animal cell as the host, preferably the recombinant vector is capable of autonomous replication within the cell and is also composed of a promoter, RNA splice site, target gene, polyadenylated site and a transcription terminating sequence. As desired, it may also contain an origin of replication. As a promoter, SRα promoter, SV 40 promoter, LTR promoter; CMV promoter and the like can be used, and early gene promoter of cytomegalovirus and the like can also be used. As a method for introducing the recombinant vector into an animal cell, preferably, for example, electroporation, the calcium phosphate technique, lipofection or the like is used.

When employing a prokaryote as the host, preferably the recombinant vector is capable of autonomous replication within the bacterium and is also composed of a promoter, a ribosomal binding sequence, the target gene and a transcription terminating sequence. It may also contain a gene that regulates the promoter.

When employing bacteria as the host, the promoter is not particularly limited and any promoter may be used as long as it can express in a host such as *Escherichia coli.* For example, a promoter derived from *Escherichia coli* or a phage, such as trp promoter, lac promoter, PL promoter or PR promoter may be mentioned. An artificially designed and modified promoter such as tac promoter may also be used. A method for introducing a recombinant vector into bacteria is not particularly limited as long as it is a method that introduces the DNA into the bacteria. Preferable examples thereof include a method using calcium ion, electroporation or the like.

When using yeast as a host, the promoter is not particularly limited and any promoter may be used as long as it can express in yeast. Examples thereof include gal1 promoter, gal10 promoter, heat shock protein promoter, MFα1 promoter, PHO5 promoter, PGK promoter, GAP promoter, ADH promoter, and AOX1 promoter. A method for introducing a recombinant vector into yeast is not particularly limited as long as it is a method that introduces the DNA into the yeast. Preferable examples thereof include a method using electroporation, spheroplast, lithium acetate or the like.

When using an insect cell as the host, preferable examples of a method for introducing a recombinant vector include a calcium phosphate technique, lipofection and electroporation.

As a specific example of the transformant of the present invention HA-ph01207#10-6 cell line may be mentioned. HA-ph01207#10-6 cell line is a cell line that was established by transfecting the CHO-K1 cell line with a vector that allows for the expression of the DNA comprising the base sequence of the ORF of the DNA represented by the base sequence described in SEQ ID NO: 1 from which a segment predicted to encode a signal sequence (20 amino acid residues from the N-terminus of the amino acid sequence represented by SEQ ID NO: 2) is excluded, as an N-terminal HA-tag fusion protein. The HA-ph01207#10-6 cell line stably expresses the N-terminal HA-tag fusion protein. A specific method for producing this cell line is described in detail in Example 2.

The HA-ph01207#10-6 cell line was deposited with the International Patent Organism Depositary of the National Institute of Advanced Industrial Science and Technology (Japan) on August 19, 2004 under Accession No. FERM BP-10101. The existence of this cell line was confirmed by experiment at the International Patent Organism Depositary on September 22, 2004.

### (Protein)

Also used is a protein encoded by the DNA used in the present invention.

As a specific form of the protein used in the present invention, for example, the protein comprising the amino acid sequence represented by SEQ ID NO: 2 of the sequence listing can be mentioned. This protein has three TSP-I domains, and also has a GPS domain and GPCR family 2 domain (seven-span transmembrane domain). The three TSP-I domains respectively comprise, in the amino acid sequence represented by SEQ ID NO: 2, the region from histidine (His) at position 297 to proline (Pro) at position 350, the region from glutamic acid (Glu) at position 352 to proline (Pro) at position 405, and the region from aspartic acid (Asp) at position 408 to proline (Pro) at position 461. The seven transmembrane domains respectively comprise, in the amino acid sequence represented by SEQ ID NO: 2, the region from valine (Val) at position 870 to phenylalanine (Phe) at position 890, the region from serine (Ser) at position 899 to glycine (Gly) at position 919, the region from valine (Val) at position 928 to leucine (Leu) at position 948, the region from arginine (Arg) at position 970 to threonine (Thr) at position 990, the region from alanine (Ala) at position 1012 to phenylalanine (Phe) at position 1032, the region from leucine (Leu) at position 1087 to alanine (Ala) at position 1107, and the region from valine (Val) at position 1114 to valine (Val) at position 1134.

Another form of the protein used in the present invention is a protein containing the amino acid sequence represented by SEQ ID NO: 2.

A further form of the protein used in the present invention is a protein having sequence homology with the present protein, and having a similarity or the like with the structural characteristics or biological function of the present protein.

A suitable sequence homology is normally a homology of 50% or more with the entire amino acid sequence, and is preferably at least 70%. More preferably, the sequence homology is more than 70%, further preferably is 80% or more, and still further preferably is 90% or more. Preferably the sequence homology in a seven-span transmembrane domain or TSP-I domain is at least 70%, preferably more than 70%, more preferably about 80% or more, and further preferably is 90% or more, and it is further preferable that these domains have a function thereof, for example, a function that localizes a protein including the domain on a membrane or an angiogenesis inhibiting function.

Examples of this kind of protein include a protein comprising an amino acid sequence having a variation including a deletion, substitution, addition or insertion of one or more, for example 1 to 100, preferably 1 to 30, more preferably 1 to 20, further preferably 1 to 10, and particularly preferably one to several amino acids in the amino acid sequence represented by SEQ ID NO: 2 and having the aforementioned biological function. The degree of variation of the amino acids and the positions and the like thereof are not particularly limited, as long as the protein having the variation has a homogeneous function to the protein comprising the amino acid sequence represented by SEQ ID NO: 2.

A protein having the variation may be a protein that was naturally produced by, for example, a mutation or posttranslational modification, or may be a protein obtained by introduction of a mutation based on a gene existing in nature. Techniques for introducing a variation are known, for example, site-directed mutagenesis, genetic homologous recombination, primer extension, and PCR, and these techniques can be used independently or in suitable combinations thereof. For example, a variation may be introduced in accordance with a method described in a book (Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.) or by modifying these methods, and Ulmer's technique (Ulmer, K. M., "Science", 1983, Vol. 219, p.666-671) can also be utilized. When introducing a variation, from the viewpoint of not altering the fundamental properties (physical properties, function, physiological activity, immunological activity or the like) of the protein, for example, mutual substitution among homologous amino acids (polar amino acids, nonpolar amino acids, hydrophobic amino acids, hydrophilic amino acids, positively charged amino acids, negatively charged amino acids and aromatic amino acids and the like) can be easily supposed.

A still further form of the protein used in the present invention can be a fragment comprising a partial amino acid sequence that is present in a designated region of the above protein. As a preferable example of this fragment, a fragment including a site at which a ligand acts in the present protein may be mentioned. A fragment including a site at which a ligand acts is useful for detecting action of ligand to the present protein, for example, for detecting binding between the present protein and the ligand, or identifying a compound that promotes or inhibits the action. Alternatively, the fragment is useful for identifying a compound having a similar action as a ligand with respect to the present protein, that is, an agonist. The fragment can also be used as an antigen in order to produce an antibody against the protein. This kind of fragment preferably comprises, as a minimum unit, five or more consecutive amino acids, more preferably eight or more, further preferably twelve or more, and still further preferably fifteen or more consecutive amino acids. These fragments can be prepared according to a known chemical synthesis method by designing a fragment having the target sequence in accordance with the amino acid sequence information of the present protein.

Although the protein used in the present invention originates in human, a protein originating in mammal that has equivalent functions and structural homology with the present protein, for example a protein derived from mouse, horse, sheep, cow, dog, monkey, cat, rat or rabbit, can also be used in the present invention.

A protein used in the present invention may be a protein prepared from a cell or biological sample in which a gene encoding the present protein was expressed by a genetic engineering technique, may be a synthetic product in a cell-free system or a product obtained by chemical synthesis, or a substance obtained by further purifying a product obtained from these. The present protein can also be a protein that expresses in a cell that includes the gene encoding the present protein. The cell can be a transformant obtained by transfection with a vector containing the gene encoding the present protein.

A constitutive amino group or carboxyl group or the like of the protein according to the present invention can also be modified to a degree that does not noticeably change the function thereof, for example, by an amidating modification or the like. Further, the protein may be one that was labeled by attaching a different protein or the like directly or indirectly via a linker peptide or the like using a genetic engineering technique or the like to the N-terminal side or C-terminal side thereof. Labeling that does not inhibit the fundamental properties of the present protein is preferable. Examples of the protein or the like to be attached include, but are not limited to, enzymes such as GST, β-galactosidase, HRP or ALP, tag peptides such as His-tag, Myc-tag, HA-tag, FLAG-tag or Xpress-tag, fluorescent substances such as fluorescein isothiocyanate or phycoerythrin, maltose-binding protein, Fc fragment of immunoglobulin and biotin. Labeling can also be carries out using radioactive isotope. The substance used for labeling can be attached individually or in combinations of two or more. By assaying the substance itself used in labeling or the function thereof, the present protein can be simply detected or purified, or, for example, interaction between the protein of the present invention and another protein can be detected.

### (Method for producing the protein)

Useful to carry out the method of the invention is a method for producing the protein of the present invention. The protein used in the present invention can be acquired, for example, by an ordinary genetic engineering technique based on the base sequence information of the gene encoding the present protein (see, Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.; Ulmer, K. M., "Science", 1983, Vol. 219, p.666-671; Ehrlich, H. A., Ed., "PCR Technology. Principles and Applications for DNA Amplification", 1989, Stockton Press). For example, the protein can be acquired by preparing a cDNA library in accordance with an ordinary method from various cells or tissue in which expression of the present DNA is confirmed or cultured cells derived from these, for example, human brain, amplifying the gene encoding the present protein using a suitable primer that is specific to the gene, and inducing expression of the obtained gene by a know genetic engineering technique.

More specifically, for example, the present protein can be produced by culturing the transformant according to the present invention, and then recovering the protein of interest from the obtained culture. Cultivation of the transformant can be carried out according to a known culture conditions and culture method that are best suited to the respective hosts. Cultivation can be carried out employing the present protein itself that is expressed by the transformant or a function thereof as an indicator. Alternatively, cultivation may be carried out employing the present protein itself or the protein amount thereof that is produced in the host or outside the host as an indicator, or by subculture or batch culture employing the amount of transformant in the culture medium as an indicator.

When the target protein expresses within the cell of the transformant or on the cell membrane, the transformant can be disrupted to extract the target protein. Further, when the target protein is secreted outside the transformant, the culture medium can be used as it is or the culture medium can be used after removing the transformant by centrifugation or the like.

As desired, the protein can be isolated and/or purified from a culture medium used to culture the transformant or from the transformant, by various isolation methods that utilize the physical properties or chemical properties thereof Isolation and/or purification can be carried out employing a function of the present protein as an indicator. Examples of isolation methods include, ammonium sulfate precipitation, ultrafiltration, gel chromatography, ion-exchange chromatography, affinity chromatography, high performance liquid chromatography, and dialysis, and these methods may be used independently or in suitable combinations thereof. Preferably, as a recommended method, an antibody that is specific to the protein of interest is prepared based on amino acid sequence information of the present protein to carry out specific adsorption using the antibody, for example, affinity chromatography utilizing a column that binds the antibody.

A protein used in the present invention can also be produced according to an ordinary chemical synthesis method. For example, solid phase synthesis, solution phase synthesis and the like are known as methods of chemically synthesizing a protein, and any of these methods can be used. These kinds of protein synthesis methods more specifically include a so-called stepwise elongation method that sequentially binds each amino acid, one at a time, to elongate a chain based on the amino acid sequence information, and a fragment condensation method that previously synthesizes fragments comprising several amino acids and subsequently subjects the respective fragments to a coupling reaction, and synthesis of the present protein can be performed by either of these methods. A condensation method used for the above described protein synthesis can also be carried out according to an ordinary method, and examples thereof include an azide method, mixed anhydride method, DCC method, active ester method, oxidation-reduction method, DPPA (diphenylphosphoryl azide) method, DCC + additive (1-hydroxybenzotriazole, N-hydroxysuccinamide, N-hydroxy-5-norbomane-2,3-dicarboxyimide and the like) method, and Woodward's method. A protein obtained by chemical synthesis can be suitably purified in accordance with various kinds of common purification methods as described above.

A protein used in the present invention can be fragmented by cleaving the protein using a suitable peptidase, and as a result, fragments of the protein can be obtained.

### (Antibody)

Also used in the present invention is an antibody against the protein used in the present invention. The antibody is produced using the present protein as an antigen. The antigen may be the present protein or a fragment thereof, and consists of at least eight, preferably at least ten, more preferably at least twelve and further preferably fifteen or more amino acids. In order to produce an antibody that is specific to the present protein and/or a fragment thereof, a region comprising a characteristic amino acid sequence of the present protein or a fragment thereof is preferably used. The amino acid sequence of this region need not necessarily be homologous or identical to a sequence of the present protein or a fragment thereof, and a site that is exposed outward on the tertiary structure thereof is preferable, and even if the amino acid sequence of the exposure site is not continuous on the primary structure, it is sufficient if the amino acid sequence is continuous with respect to the exposure site. The antibody is not particularly limited as long as it can specifically bind to or recognize the present protein and/or a fragment thereof immunologically. The presence or absence of this binding or recognition can be determined by a known antigen-antibody binding reaction.

A known antibody producing method can be utilized for production of the antibody. For example, the antibody can be obtained by administering the antigen to an animal independently or bound to a carrier in the presence or absence of an adjuvant, and performing immunological induction such as humoral response and/or cellular response. A carrier is not particularly limited as long as it does not itself exhibit an adverse action against the host and enhances antigenicity, and examples thereof include cellulose, polymeric amino acids, albumin and keyhole limpet hemocyanin. Examples of the adjuvant include Freund's complete adjuvant (FCA), Freund's incomplete adjuvant (FIA), Ribi (MPL), Ribi (TDM), Ribi (MPL + TDM), Bordetella pertussis vaccine, muramyldipeptide (MDP), aluminium adjuvant (ALUM), and combinations of these. As an animal to be immunized, mouse, rat, rabbit, goat, horse or the like is preferably used.

A polyclonal antibody can be acquired from serum of an animal that was administered with the antigen by a known antibody recovery method. As a preferable example of an antibody recovery method, immunoaffinity chromatography may be mentioned.

A monoclonal antibody can be produced by recovering antibody-producing cells (for example, lymph cells derived from spleen or lymph nodes) from an animal that was administered with the antigen, and introducing transforming means that uses known permanently proliferating cells (for example, myeloma strain of the P3-X63-Ag8 line). For example, antibody-producing cells and permanently proliferating cells are fused by a known method to produce a hybridoma, the hybridoma is cloned to screen for a hybridoma that produces an antibody that specifically recognizes the protein used in the present invention, and the antibody is then recovered from culture solution of that hybridoma.

A polyclonal antibody or monoclonal antibody obtained in this manner that can recognize and bind with the protein of the present invention can be utilized as an antibody for purification of the present protein, reagent or labeling marker. In particular, an antibody that inhibits the function of the present protein, or an antibody that binds to the present protein and exhibits a ligand-like action for the present protein can be used for functional regulation of the . present protein. These antibodies are useful for elucidating, inhibiting, improving and/or treating various kinds of diseases attributable to an abnormality in the present protein and the function thereof.

### (Identification of ligand)

Also described is a method of identifying a ligand of the protein according to the present invention. Since the present protein is a novel membrane protein receptor and a cellular biological response could be induced from HeLa cell culture supernatant when expressed in animal cell, it is considered that a ligand of the receptor is present in HeLa cell culture supernatant.

A feature of the method of identifying a ligand is contacting the substance to be examined (hereunder, referred to as "test substance") with the protein of the present invention. The protein used in the identification method may be a protein that expressed on the cell membrane of a cell including DNA encoding the present protein. The cell may be a transformant obtained by transfection with a vector including DNA encoding the present protein.

The target ligand can be obtained by detecting binding between a test substance and the protein by a known binding assay method. Alternatively, in an identification method using cells that express the present protein, the target ligand can be obtained by determining the biological response of the cells that is induced when the test substance is contacted with the protein. When the biological response of the cell when the test substance is contacted with the protein changed (was promoted, occurred, decreased, or disappeared) in comparison to the case of no contact, it can be determined that the test substance is a ligand or includes a ligand. Specific examples of the biological response of the cell include a change in cell membrane potential or a change in intracellular calcium concentration. Measurement of cell membrane potential or intracellular calcium concentration can be carried out by a known method. Alternatively, in an identification method using cells that express the present protein, the target ligand can be obtained by measuring the interaction between the present protein and a MAGUK family protein employing biological response as an indicator. When the interaction between the present protein and a MAGUK family protein in the cell when the test substance is contacted with the protein changed (was promoted or occurred) in comparison to the case of no contact, it can be determined that the test substance is a ligand or includes a ligand. Interaction between the present protein and a MAGUK family protein can be detected by a known method such as immunoblotting.

As a test substance which may be an object for identifying a ligand, for example, a sample prepared from a cell or tissue in which expression of the DNA used in the present invention was observed may be mentioned. Alternatively, various compounds that were derived from natural products or synthesized can be taken as an object.

This kind of identification method is useful for determining whether or not a ligand is included in a sample. The identification method can also be effectively used in a process for purifying the ligand from a sample which was determined to contain the ligand. For example, when fractionating and purifying a sample using gel filtration chromatography or the like, it is possible to determine whether or not a ligand is included in the fraction product.

### (Ligand)

In the present invention it was found by the above described identification method that CCK-8S (SEQ ID NO: 14) acts as a ligand of the membrane protein receptor of the present invention. More specifically, an increase in intracellular calcium concentration by action of CCK-8S (SEQ ID NO: 14) was observed in the above described HA-ph01207# 10-6 cell line that stably expressed the DNA of the present invention (see Example 5). The degree of increase in intracellular calcium concentration in the cell line caused by 1nM of CCK-8S (SEQ ID NO: 14) was roughly equal to that caused by calcium ionophore A23187 as a positive control. In CHO-K1 cell line cells that did not express the DNA of the present invention, this kind of increase in intracellular calcium concentration caused by CCK-8S (SEQ ID NO: 14) was not observed. Meanwhile, HA-ph01207#10-6 cell line did not respond to a peptide (CCK-8 Nonsulfated form, hereunder referred to as "CCK-8NS") in which the seventh tyrosine residue from C-terminus was not sulfated even though the peptide was a CCK octapeptide consisting of the same amino acid sequence as CCK-8S (SEQ ID NO: 14). Further, HA-ph01207#10-6 cell line did not respond to a tetrapeptide (CCK-4) consisting of the amino acid residues up to fourth residue from the C-terminus of CCK-8S (SEQ ID NO: 14). More specifically, an increase in intracellular calcium concentration caused by 1 nM of CCK-8NS or 1 nM of CCK-4 was not observed. It was thus clarified that HA-ph01207#10-6 cell line responds specifically to CCK-8S (SEQ ID NO: 14) and functions as a membrane protein receptor. Furthermore, because HA-ph01207#10-6 cell line responded to CCK-8S (SEQ ID NO: 14) but did not respond to CCK-8NS, the inventors considered that the fact that the seventh tyrosine residue from the C-terminus of the amino acid sequence of CCK-8S (SEQ ID NO: 14) is sulfated is important for the ligand action of CCK-8S (SEQ ID NO: 14).

Thus, since the low concentration of 1 nM of CCK-8S (SEQ ID NO: 14) induced a biological response of HA-ph01207#10-6 cell line, the inventors considered that, *in vivo,* CCK-8S (SEQ ID NO: 14) actually induces a biological response of cells through the protein encoded by the DNA used in the present invention. That is, the inventors considered that CCK-8S (SEQ ID NO: 14) is one of the *in vivo* ligands of the functional membrane protein receptor used in the present invention that was predicted to be a GPCR. Further, the membrane protein receptor used in the present invention exhibited a biological response (ligand response) including a change in membrane potential in *Xenopus laevis* oocyte when HeLa cell culture supernatant was used as a ligand source.

In addition to CCK-8S (SEQ ID NO: 14), a peptide comprising an amino acid sequence having a substitution of one homologous amino acid in the amino acid sequence of CCK-8S (SEQ ID NO: 14) and having an equivalent function to CCK-8S can also be used as ligand of the membrane protein receptor used in the present invention. The phrase "equivalent function to CCK-8S" refers to a function that induces a biological function of the membrane protein receptor of the present invention, namely, to a function that induces a biological response such as a rise in intracellular calcium concentration or a change in membrane potential in a cell expressing the membrane protein receptor usable in the present invention. Since the fact that the seventh tyrosine residue from the C-terminus of CCK-8S (SEQ ID NO: 14) is sulfated is considered important for the ligand action of CCK-8S (SEQ ID NO: 14), the sulfated tyrosine residue is retained in the ligand of the membrane protein receptor used in the present invention. A protein having the substitution may be a protein that was naturally produced by, for example, a mutation or posttranslational modification, or may be a protein obtained by introduction of a substitution based on a gene existing in nature. Techniques for introducing a substitution are known, and the above described methods can be used. From the viewpoint of not altering the fundamental properties (physical properties, functions, physiological activity, immunological activity or the like) of the protein in question mutual substitution among homologous amino acids (polar amino acids, nonpolar amino acids, hydrophobic amino acids, hydrophilic amino acids, positively charged amino acids, negatively charged amino acids and aromatic amino acids and the like) can be easily supposed. Further, CCK-8S (SEQ ID NO: 14), or peptides including a peptide comprising an amino acid sequence having a substitution of one amino acid with a homologous amino acid in the amino acid sequence of CCK-8S (SEQ ID NO: 14) and having a function that induces an increase of an intracellular calcium concentration or a change in a cell membrane potential in a cell expressing the above indicated DNA are included in the scope of the ligand of the membrane protein receptor of the present invention. In this kind of peptide, the sulfated tyrosine residue that is present at the seventh position from the C-terminus of CCK-8S is retained.

Two kinds of GPCRs, CCK-A receptor (also referred to as "CCK1 receptor") and CCK-B receptor (also referred to as "CCK2 receptor"), have been reported as CCK receptors (Herranz, R., "Medicinal Research Reviews", 2003, Vol. 23, No. 5, pp. 559-605, Review). These are both GPCRs belonging to class A (rhodopsin like). CCK-A receptor strongly expresses principally in digestive organs, and its expression is observed in one part of brain. The ligand affinity of CCK-A receptor is strong in the order of CCK-8S >> CCK-8NS, gastrin > CCK-4. Although CCK-A receptor responds strongly to CCK-8S (SEQ ID NO: 14), specificity of CCK-A receptor to the ligand is not observed. CCK-B receptor expresses widely in brain as well as digestive organs. The ligand affinity of CCK-B receptor is strong in the order of CCK-8S ≥ CCK-8NS, gastrin > CCK-4, indicating that it has lower selectivity than CCK-A receptor.

The membrane protein receptor of the present invention, which has high expression distribution specifically in brain in comparison to CCK-A receptor which has low expression in brain, is thought to participate mainly in action of CCK in the central nervous system. Further, because it has a different ligand affinity than that of CCK-B receptor for which expression in brain is high, there is a possibility that the membrane protein receptor used in the present invention exhibits a different activity to CCK-B receptor in the brain. It is therefore considered that the membrane protein receptor used in the present invention participates as a CCK receptor in physiological functions such as analgesia, sedation, ingestion suppression, memory and learning, in which CCK involvement has already been postulated. Furthermore, it is reported that CCK exhibits various actions in digestive organs, and it is also considered to be a signaling substance that imparts a sensation of satiety to cerebral neurons. It is therefore considered that CCK-8S, a member of the CCK family, also acts as a signaling substance that imparts a sensation of satiety to cerebral neurons. It is considered that a quantitative and functional decline in CCK-8S causes obesity due to a decline in a sensation of satiety. It is thought that the membrane protein receptor of the present invention is involved in this kind of obesity as a CCK receptor. Further, it is reported that when CCK-8S is administered to diabetes patients, an increase in insulin amounts is promoted and an increase in postcibal glucose amount is suppressed (Bo, A. et al., "The Journal of Clinical Endocrinology & Metabolism", 2000, Vol. 85, pp. 1043-1048). There is thus a possibility that the functional membrane protein receptor used in the present invention is associated with diabetes. Accordingly, an agonist or antagonist of the membrane protein receptor used in the present invention can alleviate a disease or symptoms accompanying impairment of this kind of physiological function. More specifically, an agonist or antagonist of the membrane protein receptor used in the present invention can be used as an active ingredient of an antianxiety drug, analgesic preparation or preventive and/or therapeutic agent for a disease caused by various kinds of abnormalities of the central nervous system. Specific examples of these kinds of diseases include dementia, Parkinson's disease, panic syndrome, drug dependence, obesity, diabetes and the like.

Since one of the ligands for the membrane protein receptor of the present invention is, as described above, CCK-8S (SEQ ID NO: 14), it is considered that the membrane protein receptor used in the present invention is involved in neurologic functions such as memory retention, in which CCK-8S (SEQ ID NO: 14) is thought to participate. A decline in the quantity and/or function of CCK-8S (SEQ ID NO: 14) or the disappearance thereof causes the appearance of pathologic symptoms such as difficulty in recalling memory to a conscious level and translation into action. Diseases or symptoms accompanying this kind of impairment of memory function can be alleviated by an agonist of the membrane protein receptor used in the present invention. Examples of this kind of disease include diseases accompanying impairment of neurologic function such as memory, and as specific examples, dementia and Alzheimer's disease and the like may be mentioned.

### (Method of Identifying Compound)

An aspect of the present invention relates to a method of identifying an agonist of the membrane protein receptor of the present invention. As a method of identifying an agonist of the membrane protein receptor of the present invention, for example, a method may be mentioned which comprises, in a test system using a transformant that expressed the present protein, measuring a change in functions generated in the transformant after contacting the transformant and a compound to be examined (hereunder, referred to as "test compound"), or in the presence of the test compound. By detecting a change, such as a reduction, increase, disappearance, or appearance, in a function of the membrane protein receptor used in the present invention in comparison with a measurement result obtained in the absence of the test compound, it is possible to select an agonist of the membrane protein receptor used in the present invention. More preferably, an agonist can be selected by measuring a functional change in the membrane protein receptor of the present invention caused by a ligand of the membrane protein receptor, for example, CCK-8S (SEQ ID NO: 14), and comparing the functional change. Preferably, the agonist is a compound that brings about a functional change in the membrane protein receptor used in the present invention that is equivalent with a functional change produced in the membrane protein receptor by a ligand, for example, CCK-8S (SEQ ID NO: 14). It is sufficient that a functional change produced in the membrane protein receptor used in the present invention by an agonist is equivalent to a functional change produced in the membrane protein receptor of the present invention by a ligand, for example, CCK-8S (SEQ ID NO: 14), and there may be a quantitative difference. For example, a functional change produced in the membrane protein receptor used in the present invention by an agonist may be weaker than a functional change produced in the membrane protein receptor of the present invention by a ligand, for example, CCK-8S (SEQ ID NO: 14). Preferably, an agonist that induces an equal functional change is selected. The functional change of the membrane protein receptor used in the present invention can be measured by employing a change in biological response via the membrane protein receptor of a transformant as an indicator. Accordingly, as a functional change that is equivalent with a functional change produced in the membrane protein receptor used in the present invention by a ligand, for example, CCK-8S (SEQ ID NO: 14), for example, an increase in intracellular calcium concentration via the membrane protein receptor in a transformant can be mentioned. Measurement of a change in intracellular calcium concentration can be carried out using a known method (see Example 5). In addition, as a functional change that is equivalent with a functional change produced in the membrane protein receptor used in the present invention by a ligand, for example, CCK-8S (SEQ ID NO: 14), for example, a change in membrane potential via the membrane protein receptor in a transformant can be mentioned. Measurement of a change in membrane potential can be carried out using a known method (see Example 3). With respect to the ligand, either a sample including the ligand or the ligand itself that was obtained by the above described method of identifying a ligand can be used. Since it is considered that CCK-8S (SEQ ID NO: 14) is an *in* vivo ligand of the present protein, the use of CCK-8S (SEQ ID NO: 14) as a ligand is preferred. CCK-8S (SEQ ID NO: 14) can be produced by a common chemical synthesis method. It can also be synthesized using a commercially available peptide synthesis apparatus.

The method of identifying an agonist of the membrane protein receptor used in the present invention can also be carried out by determining whether or not a compound obtained by a method for identifying a compound that binds with the membrane protein receptor of the present invention induces a functional change in the membrane protein receptor of the present invention using the above described identification method.

A method for identifying a compound that binds with the membrane protein receptor used in the present invention can be carried out according to a method in which the protein used in the present invention and a ligand of the protein are reacted in the presence and absence of a test compound to measure binding between the protein and the ligand. A protein used in the present identification method can be a protein that expressed on the cell membrane of a cell containing DNA encoding the present protein. The cell may be a transformant obtained by transfection with a vector containing DNA encoding the present protein. In the present identification method, a compound that inhibits binding between the present protein and the ligand may be a compound that binds with the present protein or a compound that binds with the ligand. Measurement of binding between the present protein and a ligand of the protein can be performed utilizing various kinds of binding assays that are used in an ordinary pharmaceutical screening system. For example, measurement of binding can be carried out by performing a binding reaction between the ligand and the present protein, and then assaying the ligand that bound to the present protein using an anti-ligand antibody. The anti-ligand antibody that bound to the ligand can be detected using a secondary antibody labeled with HRP or biotin or the like. The ligand that bound to the present protein can also be detected using an anti-ligand antibody that was previously labeled with HRP or biotin or the like. Alternatively, the ligand that bound to the present protein can be assayed by performing the above described identification method using a ligand that was previously labeled with a desired labeling substance as the ligand for use in the binding reaction with the present protein, and then detecting the labeling substance. Any substance that is used in an ordinary binding assay can be utilized as the labeling substance. As a simple and convenient method, a radioactive isotope can be utilized. By determining the action of a compound obtained by this kind of identification method with respect to the biological response of a transformant as described above, it is possible to determine whether or not the compound induces a functional change in the membrane protein receptor used in the present invention. If a functional change in the membrane protein receptor used in the present invention produced by the compound is equivalent with a functional change produced in the membrane protein receptor used in the present invention by a ligand, for example, CCK-8S (SEQ ID NO: 14), it can be determined that the compound is an agonist that binds to the membrane protein receptor used in the present invention and induces a biological response via the membrane protein receptor. In contrast, when a functional change in the membrane protein receptor used in the present invention is not produced by the compound, it can be determined that the compound is an antagonist that binds to the membrane protein receptor used in the present invention but does not induce a biological response via the membrane protein receptor, or is a compound that acts on a ligand to inhibit binding between the ligand and the membrane protein receptor.

A further aspect of the present invention relates to a method of identifying a compound that inhibits or promotes the function of a protein according to the present invention. The method of identifying a compound that inhibits or promotes the function of the present protein can be carried out utilizing a known pharmaceutical screening system using at least one member selected from the group consisting of the present protein, DNA, recombinant vector, transformant, or antibody. According to the preset identification method, it is possible to conduct screening for an antagonist by drug design based on the conformation of the protein, screening for an inhibitor or promoter of expression at the gene level utilizing a protein synthesis system, or screening for an antibody-recognizing substance utilizing the antibody.

Identification of a compound that inhibits or promotes a function of a protein used in the present invention can be carried out, for example, by allowing the protein and the test compound to coexist in a test system that can measure the function of the present protein under conditions that enable interaction between the protein and the test compound, measuring the functions thereof, and then detecting a change, such as a decrease, increase, disappearance or appearance, in the functions of the protein in comparison to a measurement result obtained in the absence of the test compound. Since the protein in question is a membrane protein receptor, as examples of a function of the protein, binding with a ligand, activation of an intracellular signal transduction mechanism, and induction of cellular response may be mentioned. More specific examples include binding with CCK-8S (SEQ ID NO: 14) or interaction with a MAGUK family protein and the like. A function of the protein can also be measured by employing a biological response of a cell expressing the protein, for example, a change in cell membrane potential or a change in intracellular calcium concentration, as an indicator. Measurement of a function of the protein can also be performed by directly detecting the function, or can be carried out, for example, by introducing a signal as an indictor of the function into a test system and detecting the signal. As a signal, a tag peptide such as GST, His-tag, Myc-tag, HA-tag, FLAG-tag or Xpress-tag, or a fluorescent substance or the like can be used, and any labeling substance can be used as long as it is a substance that is used in an ordinary method for identifying a compound.

The effect exerted on a function of a protein used in the present invention by the test compound can be determined by comparing the function of the present protein in a case where it was allowed to coexist with the test compound and the function of the present protein in a case where it was not allowed to coexist with the test compound. When the function of the present protein in the case where it was allowed to coexist with the test compound decreased in comparison with the function of the present protein in the case where it was not allowed to coexist with the test compound, it can be determined that the test compound has an action that inhibits the function of the present protein. In contrast, when the function of the present protein in the case where it was allowed to coexist with the test compound increased in comparison with the function of the present protein in the case where it was not allowed to coexist with the test compound, it can be determined that the test compound has an action that promotes or stabilizes the function of the present protein.

A further aspect of the present invention relates to a method of identifying a compound that promotes or inhibits binding between a protein according to the present invention and a ligand of the protein. The identification method can be carried out according to a method in which the protein of the present invention and a ligand of the protein are reacted in the presence and absence of a test compound to measure binding between the protein and the ligand. Other examples of the identification method include a method which measures binding between the protein and the ligand in a similar manner using a cell expressing the present protein or a transformant that expressed the present protein, in place of the present protein. According to this kind of identification method, it is possible to identify a compound that inhibits binding between the present protein and a ligand of the protein or a compound that promotes the binding. A compound that inhibits binding between the present protein and the ligand may be a compound that binds with the present protein or a compound that binds with the ligand. Since the present protein is a membrane protein receptor, a compound that inhibits binding between the present protein and the ligand of the protein may be a compound that inhibits a function of the membrane protein receptor or a compound that promotes such function. Whether the compound is a compound that inhibits a function of the membrane protein receptor used in the present invention or a compound that promotes such function can be determined by measuring a change in the function of the membrane protein receptor used in the present invention caused by a ligand in the presence and absence of the compound. A compound that promotes binding between the present protein and the ligand may be a compound that promotes a function of the membrane protein receptor used in the present invention. It is possible to determine that the compound is a compound that promotes a function of the membrane protein receptor used in the present invention by measuring a change in the function of the membrane protein receptor used in the present invention caused by a ligand in the presence and absence of the compound.

As a method for measuring a function of a protein used in the present invention, an experimental method using a transformant that expressed the present protein may be mentioned which comprises allowing a ligand to act with respect to the protein after contacting the transformant and a test compound, or in the presence of the transformant and the test compound, and then measuring a change in a biological response produced in the transformant. This kind of measurement method can be utilized to carry out a method of identifying a compound that promotes or inhibits a function of the present protein. As a ligand that is allowed to act on the protein, either a sample including the ligand or the ligand itself that was obtained according to the above described method of identifying a ligand can be used. Since it is considered that CCK-8S (SEQ ID NO: 14) is an *in vivo* ligand of the present protein, the use of CCK-8S (SEQ ID NO: 14) as a ligand is preferred. By detecting a change, such as a decrease, increase, disappearance, or appearance, in function in comparison with a measurement result obtained in the absence of the test compound, it is possible to select a compound that inhibits or promotes the function of the present protein. Examples of a change in a biological response produced in a transformant that expressed the present protein include a change in cell membrane potential or a change in intracellular calcium concentration and the like. When a change in the cell membrane potential of the transformant is amplified by a compound or a change such as an increase in the intracellular calcium concentration was produced by the compound, it can be determined that the compound promotes the function of the present protein. Conversely, when a change in the cell membrane potential of the transformant is decreased by a compound or a change such as a decrease in the intracellular calcium concentration was induced by the compound, it can be determined that the compound inhibits the function of the present protein. Measurement of a change in cell membrane potential or a change in intracellular calcium concentration can be carried out using a known method. Further, measurement of a function of the present protein can be carried out by measuring interaction with a MAGUK family protein. Measurement of a change in interaction with a MAGUK family protein or measurement of a change in binding with a G protein can be carried out using a known method.

A still further aspect of the present invention relates to a method of identifying a compound that can affect interaction between a protein used in the present invention and a MAGUK family protein. Interaction between the present protein and a MAGUK family protein can be measured, for example, by expressing the present protein according to a genetic engineering technique and then detecting binding thereof with a MAGUK family protein. More specifically, a MAGUK family protein is, for example, expressed as a GST-tag fusion protein according to a genetic engineering technique, then bonded to glutathione-sepharose, after which the amount of the present protein binding thereto can be quantified using an antibody against the present protein, for example an antibody that was labeled with an enzyme such as HRP or ALP, a radioactive isotope, a fluorescent substance, or biotin or the like. When the present protein is fused with a tag peptide and used, the amount of binding can be determined using an anti-tag antibody. Naturally, the present protein may also be directly labeled with the above described enzyme, radioactive isotope, fluorescent substance, or biotin or the like. Alternatively, a secondary antibody that was labeled with the above described enzyme, radioactive isotope, fluorescent substance, or biotin or the like may be used. As a further alternative, DNA encoding the present protein and DNA encoding a MAGUK family protein may be co-expressed using a suitable cell, whereby the interaction of the two substances can be measured by detecting binding between the two substances using a pull-down method.

A method of identifying a compound that affects interaction between a protein used in the present invention and a MAGUK family protein can also be carried out, for example, by using a two-hybrid method to introduce into a yeast or a eukaryotic cell or the like a plasmid expressing the present protein and a DNA binding protein as a fusion protein, a plasmid expressing a MAGUK family protein and a transcription-activating protein as a fusion protein, and a plasmid containing a reporter gene such as lacZ connected to an appropriate promoter gene, and comparing the expression amount of the reporter gene when allowed to coexist with the test compound and the expression amount of the reporter gene in the absence of the test compound. When the expression amount of the reporter gene when allowed to coexist with the test compound decreased in comparison to the expression amount of the reporter gene in the absence of the test compound, it can be determined that the test compound has an action that inhibits binding between the present protein and the MAGUK family protein. In contrast, when the expression amount of the reporter gene when allowed to coexist with the test compound increased in comparison to the expression amount of the reporter gene in the absence of the test compound, it can be determined that the test compound has an action that stabilizes binding between the present protein and the MAGUK family protein.

Identification of a compound that affects interaction between a protein used in the present invention and a MAGUK family protein can also be performed using a surface plasmon resonance sensor such as the BIACORE system.

Further, identification of a compound that affects interaction between a protein used in the present invention and a MAGUK family protein can be carried out using a method that applies a scintillation proximity assay (SPA) or fluorescence resonance energy transfer (FRET).

Specific examples of a MAGUK family protein used in the identification method according to the present invention include DLG2, DLG3 and DLG4, or AIP1 and MAGI3. As long as there is no affect on the interaction with the protein of the present invention, the MAGUK family protein may be one in which a segment thereof was deleted or one to which a labeling substance such as another protein was attached.
Described herein is a method of identifying a compound that inhibits or promotes expression of a DNA used in the present invention. The identification method can be carried out by allowing the DNA and a test compound to coexist in a test system in which it is possible to measure expression of the DNA, measuring the expression, and then detecting a change, such as a decrease, an increase, disappearance or appearance, in the expression in comparison to a measurement result obtained in a case without coexistence of the test compound. Measurement of the expression of the protein can be performed by directly detecting a protein coded for by the DNA, or can be carried out, for example, by introducing a signal as an indictor of the expression into a test system and detecting the signal. As a signal, a tag peptide such as GST, His-tag, Myc-tag, HA-tag, FLAG-tag or Xpress-tag, or a fluorescent substance or the like can be used.

Identification of a compound that affects expression of the DNA used in the present invention can also be carried out by, for example, producing a vector in which a reporter gene is connected instead of the DNA downstream of a promoter region of a gene including the present DNA, contacting a cell, for example, a eukaryotic cell, containing the vector with the test compound, and determining the presence or absence of, or a change in, expression of the reporter gene. A gene that is ordinarily used in a reporter assay can be used as the reporter gene, and for example, a gene having an enzyme activity such as luciferase, β-galactosidase or chloramphenicol acetyl transferase may be mentioned. Detection of expression of the reporter gene can be carried out by detecting the activity of the gene product, for example, in the case of the reporter genes exemplified above, the enzyme activity.

Identification of a compound that affects expression of the DNA used in the present invention can also be carried out by contacting a transformant used in the present invention and a test compound in a test system that expresses a protein using the transformant, and then measuring the expressed protein. A compound that inhibits or promotes expression of the present DNA can be selected by detecting a change, such as a decrease, increase, disappearance or appearance, in the expression in comparison with a measurement result obtained in the absence of the test compound. Detection of the presence or absence of, or a change in, expression of the protein can be simply and conveniently carried out by employing as an indicator a biological function of the protein to be expressed or a biological response of a cell via the protein, for example, interaction with a MAGUK family protein, a change in cell membrane potential, or a change in intracellular calcium concentration that is produced when a ligand is allowed to act thereon.

### (Compound)

Compounds identified in the above manner can be used as agonists of the membrane protein receptor used in the present invention. Further, compounds acquired by the identification methods as described above can be utilized as inhibitors, antagonists, promoters or stabilizers or the like of a function of the protein used in the present invention, for example, binding with a ligand, activation of intracellular signal transduction, or induction of a cellular response. Furthermore, the compounds can also be utilized as expression inhibitors or expression promoters with respect to the above protein at the gene level. These compounds can be prepared as medicines by performing further screening which takes into consideration the balance between biological usefulness and toxicity. Further, it can be anticipated that these compounds will produce a preventive effect and/or therapeutic effect for various kinds of pathologic symptoms attributable to an abnormality in a function of the present protein and/or expression of DNA encoding the protein.

### (Pharmaceutical Composition)

The protein, DNA, recombinant vector, transformant, antibody, ligand and compound used in the present invention is useful as an active ingredient of a medicine or pharmaceutical composition that is based on inhibiting, antagonizing, or promoting a function and/or expression of the present protein.

The inventors found that tissue expression of DNA comprising the base sequence represented by SEQ ID NO: 1 of the sequence listing as one form of the DNA used in the present invention is specifically high in the overall brain, for example, temporal pole, motor cortex, and orbital gyri. Accordingly, the inventors considered that the protein used in the present invention, the DNA and the protein are important for homeostatic maintenance of the brain and brain cells. Further, expression thereof that is noticeably high in comparison to normal tissue has been observed in tumors such as ovarian cancer, liver cancer, and adrenal cancer. The inventors therefore considered that the present protein, the DNA and the protein are involved in these tumor diseases.

A protein used in the present invention has a TSP-I domain in the amino acid sequence thereof. Since it is reported that the TSP-I domain is a domain that is responsible for an angiogenesis inhibiting function, the inventors considered that the present protein has an angiogenesis inhibiting function. Accordingly, there is a possibility that the present protein is involved in diseases caused by angiogenesis inhibition or diseases accompanying angiogenesis inhibition. A function or expression of the present protein is preferably inhibited in diseases which can be treated by promoting angiogenesis. Examples of these diseases include cerebral contusion and cerebral infarction. In this case, improvement, prevention and/or treatment of the disease can be carried out by a compound having a function that reduces or eliminates a function and/or expression of the present protein. In contrast, there is a possibility that a decrease in a function and/or expression of the present protein is associated with diseases caused by angiogenesis or diseases accompanying angiogenesis. Examples of these diseases include tumor diseases that are known to accompany angiogenesis. In such case, improvement, prevention, and/or treatment can be carried out using the present protein, the DNA or a compound that promotes a function of the protein and/or expression of the DNA. More specifically, a pharmaceutical composition comprising an effective dose of the present protein, the DNA or a compound that promotes a function of the protein and/or expression of the DNA can be used as improving agent, preventive agent, and/or therapeutic agent for a disease caused by angiogenesis or a disease accompanying angiogenesis (for example, a tumor disease). Further, a method for preventing and/or a method for treating such disease can be carried out using these.

It is reported that CCK-8S (SEQ ID NO: 14) that was found as a ligand of the functional membrane protein receptor used in the present invention is essential for memory retention, for example, that it is difficult to recall memory to a consciousness level and translate it into action in the absence of CCK-8S (SEQ ID NO: 14). The inventors therefore considered that the functional membrane protein receptor used in the present invention is involved in the neurologic function of CCK-8. Further, since a relationship between CCK-B receptor and anxiety has been reported, there is a possibility that the functional membrane protein receptor used in the present invention is similarly related with anxiety disorder. Accordingly, the inventors considered that an agonist of the functional membrane protein receptor of the present invention and a pharmaceutical composition comprising an effective dose of the agonist is effective in alleviating, improving, preventing and/or treating diseases or symptoms accompanying impairment of neurologic functions such as memory as well as anxiety disorders and the like. Specific examples of diseases accompanying impairment of neurologic functions such as memory include dementia (including Alzheimer's disease). Furthermore, it is reported that CCK exhibits various kinds of actions in digestive organs, and it is also considered to be a signaling substance that imparts a sensation of satiety to cerebral neurons. The inventors therefore considered that CCK-8S, a member of the CCK family, also acts as a signaling substance that imparts a sensation of satiety to cerebral neurons. A quantitative and functional decrease in CCK-8S is considered to bring about obesity due to a decrease in a sensation of satiety. Further, it is reported that when CCK-8S is administered to diabetes patients, an increase in insulin amount is promoted and an increase in postcibal glucose amount is suppressed (Bo, A. et al., "The Journal of Clinical Endocrinology & Metabolism", 2000, Vol. 85, pp. 1043-1048). There is thus a possibility that the functional membrane protein receptor used in the present invention is associated with diabetes. Accordingly, the inventors considered that an agonist of the functional membrane protein receptor used in the present invention and a pharmaceutical composition comprising the agonist are effective in improving, preventing, and/or treating diabetes or obesity. More specifically, an agonist of the functional membrane protein receptor used in the present invention and a pharmaceutical composition comprising the agonist can be used as improving agents, preventive agents and/or therapeutic agents for a disease or symptoms accompanying impairment of memory function (for example, dementia (including Alzheimer's disease)), as well as diabetes and obesity. Further, a method for preventing and/or method for treating such disease can be carried out using these.

According to the present invention, for example, by utilizing the base sequence of all or a part of the DNA of this invention, it is possible to specifically detect the existence or non-existence of an abnormality in a gene that includes the DNA in an individual or in various kinds of tissue, or to detect the existence or non-existence of expression of the gene. By detecting the DNA, it is possible to perform diagnosis of susceptibility to, onset and/or prognosis of a disease attributable to the gene. The phrase "disease attributable to the gene" refers to a disease that is attributable to a quantitative abnormality and/or functional abnormality or the like of the gene. It is known that CCK-8S (SEQ ID NO: 14) that was found as a ligand of the membrane protein receptor of the present invention is involved in neurologic functions such as memory. Accordingly, when an abnormality is produced in the signal transduction from CCK-8S (SEQ ID NO: 14) that is caused by an abnormality of the function of the membrane protein receptor as the gene product of the gene used in the present invention due to a quantitative abnormality and/or functional abnormality in the gene, there is a possibility of occurrence of a disease or symptoms that accompany impairment of a neurologic function such as memory. Thus, examples of a disease attributable to the present gene include diseases or symptoms that accompany impairment of a neurologic function such as memory, for example, Alzheimer's disease. Further, the protein used in the present invention has a TSP-I domain in the amino acid sequence thereof. Since it is reported that the TSP-I domain is a domain that is responsible for an angiogenesis inhibiting function, the present protein may be involved in a disease attributable to angiogenesis inhibition or a disease accompanying angiogenesis inhibition, or, alternatively, a disease attributable to angiogenesis or a disease accompanying angiogenesis. Examples of such kinds of disease include a tumor disease such as a brain tumor, ovarian cancer, liver cancer, and adrenal cancer, as well as cerebral contusion or cerebral infarction.

Diagnosis of a disease by gene detection can be carried out, for example with respect to a test sample, by detecting the presence of nucleic acids corresponding to the gene, determining the existing amount thereof and/or identifying a mutation. By comparison with a normal control sample, an alteration in the existence of nucleic acids corresponding to the gene of interest and a quantitative alteration thereof can be detected. Further, by comparison with a normal genotype it is possible to detect, for example, a deletion and insertion by measuring a size alteration with respect to an amplification product that was produced by amplifying nucleic acids corresponding to the gene of interest by a known method. Furthermore, a point mutation can be identified by hybridizing amplification DNA with, for example, DNA used in the present invention that was labeled. The above described diagnosis can be carried out by detection of an alteration or a mutation in this manner. A nucleic acid sample may be prepared according to various methods for facilitating detection of a target sequence, for example, denaturation, digestion with restriction enzyme, electrophoresis, or dot blotting.

A known gene detection method can be used as the detection method, and examples thereof include plaque hybridization, colony hybridization, Southern blotting, Northern blotting, the NASBA method and RT-PCR. Measurement at the cell level utilizing in situ RT-PCR or in situ hybridization or the like can also be used. Methods that can be used to detect the gene of the present invention are not limited to the methods described above, and any known gene detection method can be used.

For this kind of gene detection method, a DNA fragment used in the present invention that has a property as a probe or that has a property as a primer is useful in carrying out identification of the gene of interest or a mutant gene thereof, and/or amplification thereof. The phrase "DNA fragment having a property as a probe" refers to a substance comprising a characteristic sequence of the DNA of the present invention that can specifically hybridize to the DNA only. The phrase "substance having a property as a primer" refers to a substance comprising a characteristic sequence of the present DNA that can specifically amplify the DNA only. Further, when detecting a mutant gene capable of amplification, a probe or a primer having a sequence of a predetermined length that includes a location having a mutation within the gene is produced and used. In general, the length of the base sequence of the probe or the primer is preferably from about 5 to 50 nucleotides, more preferably from about 10 to 35 nucleotides, and further preferably from about 15 to 30 nucleotides. Although a labeled probe is normally used as the probe, the probe may be unlabeled, and may be detected directly or indirectly by specific binding with a labeled ligand. Various methods are known as methods for labeling a probe and a ligand, and examples thereof include methods utilizing nick translation, random priming or kinase treatment. As suitable labeling substances, a radioactive isotope, biotin, a fluorescent substance, a chemiluminescent substance, an enzyme, an antibody and the like may be mentioned.

PCR is preferable as the gene detection method from the viewpoint of sensitivity. The PCR method is not particularly limited as long as it is a method that uses a DNA fragment that can specifically amplify the gene of interest as a primer, for example, a conventional known method such as RT-PCR may be mentioned, and various modified methods that are used in the art can be applied.

In addition to detection of a gene, assay of the DNA of the gene of interest and/or a mutant gene thereof can be performed by PCR. As examples of this kind of analysis method, a competitive assay that is similar to an MSSA method, or PCR-SSCP which is known as a mutation detection method that utilizes variations in mobility accompanying changes in the conformation of single-strand DNA may be mentioned.

It is also possible to specifically detect, for example, the existence or non-existence of an abnormality in a protein according to the present invention and a function thereof in an individual or in various kinds of tissue by utilizing the protein. By detecting an abnormality in the present protein or a function thereof, it is possible to perform diagnosis of susceptibility to, onset and/or prognosis of a disease attributable to the gene.

Diagnosis of a disease by detection of a protein can be carried out, for example with respect to a test sample, by detecting the presence of the protein, determining the existing amount thereof, and/or detecting a mutation. More specifically, the present protein and/or a mutant thereof are quantitatively or qualitatively determined. By comparison with a normal control sample, an alteration in the existence of the protein of interest and a quantitative alteration thereof can be detected. In a comparison with a normal protein, for example, a mutation thereof can be detected by determining the amino acid sequence. The above described diagnosis can be carried out by detecting this kind of alteration or mutation. The test sample is not particularly limited as long as it includes the protein of interest and/or a mutant thereof. For example, the test sample may be a biological sample derived from a living organism such as blood, serum, urine, biopsy tissue or the like.

Determination of the protein used in the present invention as well as the protein having a mutation can be carried out by use of the present protein, for example, the protein comprising the amino acid sequence represented by SEQ ID NO: 2 of the sequence listing, or an amino acid sequence having a deletion, substitution, insertion or addition of from one or several to multiple amino acids in the amino acid sequence of the protein, fragments of these, or an antibody against the protein or a fragment thereof.

Quantitative or qualitative determination of the protein can be performed using a protein detection method or assay method according to a common technique in the field of the art. For example, a mutant protein can be detected by analyzing the amino acid sequence of the protein of interest, and more preferably, a variation in the sequence of the protein of interest or the existence or non-existence of the protein of interest can be detected using an antibody (a polyclonal or monoclonal antibody).

More specifically, the above detection can be carried out for a test sample by performing immunoprecipitation using a specific antibody against the protein of interest, and conducting analysis of the protein of interest by Western blotting or immunoblotting. Further, the protein of interest can be detected in a paraffin or frozen tissue section by an antibody against the protein of interest using an immunohistochemical technique.

As preferred specific examples of a method that detects the protein of interest or a mutant thereof, immuno-enzyme assay (IEMA), immunoradiometric assay (IRMA), radioimmunoassay (RIA) and enzyme-linked immunosorbent assay (ELISA) including a sandwich method using a monoclonal antibody and/or polyclonal antibody may be mentioned. In addition, a radioimmunoassay or a competitive binding assay and the like can also be utilized.

The protein, DNA, recombinant vector, transformant and antibody used in the present invention can each be used by itself as a reagent or the like. For example, each of these can be used as a reagent for use in the method of identifying a compound according to the present invention.
The reagent is useful, for example, in elucidating a cellular signal transduction pathway in which the present protein or DNA participates, as well as for fundamental research relating to diseases and the like attributable to an abnormality in the protein and/or DNA.

When these are reagents, they may include a substance such as a buffer solution, a salt, a stabilizer and/or an antiseptic agent. In this connection, known formulation means may be introduced in accordance with the respective properties at the time of formulation.

Although the present invention is described specifically by the following examples, the present invention is not limited to the following Examples.

### Example 1

### (Construction of human brain-derived cDNA library and isolation of gene)

A cDNA library was constructed according to an ordinary method employing commercially available polyA⁺ RNA derived from the human brain, fetal brain and brain hippocampus (Clontech Inc.: catalog Nos. 6516-1, 6525-1, and 6578-1) as starting material, and the base sequences of cDNA clones were determined after isolating cDNA fragments by dbEST analysis. More specifically, in accordance with the method of Ohara et al. (Ohara, O. et al., "DNA Research", 1997, Vol. 4, p. 53-59), approximately 50,000 recombinants were randomly selected from the cDNA library derived from human brain that was prepared as described above, and the base sequences at the 5'-terminus and 3'-terminus were determined for cDNA of approximately 30,000 clones among these. Further, approximately 1,100 clones were selected by mainly in-vitro transcription translation experiment, and the base sequences of the cDNA of these were determined according to the method of Ohara et al.

For cDNA clones whose entire base sequence was determined, the ORF was predicted by a generic analysis method using a computer program to obtain a cDNA clone having a seven-span transmembrane domain in this region.

The identified cDNA clone ph01207 is a DNA (SEQ ID NO: 1) having a novel base sequence of a total length of 4557 bp including an ORF comprising 1518 amino acid residues having a segment (20 amino acid residues from the N terminus) that is predicted to be a signal sequence. Based on a homology search, it is considered that ph01207 is a splice variant having a deleted region encoding 55 amino acid residues at the N-terminal region in the sequence of hBAI2 (GenBank accession number AB005298), and also having added one amino acid residue in a region on the C-terminal side (lysine at position 1406 in the amino acid sequence represented by SEQ ID NO: 2) (Figure 1).

As the structural characteristics of the protein encoded by ph01207, it was found that in addition to three TSP-I domains, the protein has a GPS domain and a GPCR family-2 domain (seven-span transmembrane domain).

Meanwhile, it is considered that the protein encoded by hBAI2 has a GPS domain and a GPCR family-2 domain, in addition to four TSP-I domains. It was thus clarified that 55 amino acid residues corresponding to a region including one TSP-I domain on the N-terminal region side of hBAI2 are deleted in the protein encoded by ph01207.

Further, in analysis of tissue in which ph01207 gene was expressed, specifically high expression of the gene was observed universally in normal brain tissue (for example, temporal pole, motor cortex, orbital gyri and the like). Further, enhancement of expression was observed in ovarian cancer, liver cancer, and adrenal cancer in comparison to the respective normal tissue.

### Example 2

### (Production of ph01207 expression cell line and expression of DNA in the cell line)

Using the clone ph01207 that was identified in Example 1, the protein encoded by ph01207 was expressed as an N-terminal epitope-tag fusion protein.

First, an expression vector containing the ph01207 gene was constructed. Using the clone ph01207 identified in Example 1, DNA encoding the amino acid sequence excluding a segment predicted to be a signal sequence (20 amino acid residues from N terminus) was cloned into pDONR201 by PCR and restriction enzyme treatment to construct a ph01207 entry vector. PCR was carried out using oligonucleotides comprising the base sequences represented by SEQ ID NOS: 4 to 11 in the sequence listing, respectively, as primers, and using Pfu turbo DNA polymerase (Stratagene) as polymerase.

Two kinds of vector, p3×FLAG-CMV9-attR and T8HA-attR/pCINeo, were prepared as N-terminal epitope-tag fusion type expression vectors. p3×FLAG-CMV9-attR is a vector obtained by making p3×FLAG-CMV9 (Sigma Inc.) compatible to the Gateway system using the Gateway Vector Conversion System (Invitrogen Corp.). T8HA-attR/pCINeo was constructed using pCINeo (Promega Corp.), synthetic oligos (T8SP-HA (SEQ ID NO: 12) and T8SP-HA as (SEQ ID NO: 13)) and the Gateway Vector Conversion System in accordance with information in the literature (Koller, K. J., et al., "Analytical Biochemistry", 1997, Vol. 250, p. 51-60). Both vectors have a secretory signal sequence (FLAG: PPTLS, HA:T8) before (N-terminal side) epitope-tag. Using these vectors, an N-terminal FLAG-tag or HA-tag fusion type expression vector was constructed by LR reaction with Mammalian Expression system with Gateway technology (Invitrogen Corp.). For each of the constructed expression vectors it was confirmed by restriction enzyme treatment and sequence analysis that no base substitution or deletion existed in a coding region in the introduced sequence.

After transfecting each of the thus constructed two kinds of expression vector to the CHO-K1 cell line using FuGENE 6 (Roche), selection of the expression cell line was performed by cultivation with a culture medium including G418 (DMEM/F12 medium containing 400 µg/mL G418 and 10% fetal calf serum). For expression cell lines that grew, selection was further performed by cell enzyme immunoassay (cell EIA) using peroxidase (POD)-labeled antibody (anti-FLAG M2-POD antibody and anti-HA-POD antibody: 3F10) against epitope-tag. For the selected cell lines, selection was further carried out by fluorocytometry (FCM) analysis using primary antibody (anti-FLAG M2 antibody and anti-HA antibody: clone HA-7) and fluorescein isothiocyanate (FITC)-labeled secondary antibody (FITC-anti-mouse IgG antibody), to obtain expression cell lines.

As the result of the FCM analysis, 8 clone lines expressing a protein (FLAG-tag fusion protein) recognized by anti-FLAG antibody on the cell membrane and 4 clone lines expressing a protein (HA-tag fusion protein) recognized by anti-HA antibody were obtained. Since a fluorescent signal produced by bonding of an antibody recognizing FLAG-tag or HA-tag was clearly stronger for each of these clones in comparison to the host cell line (CHO-K1), it was clarified that the gene of interest was expressed therein. Representative results are shown in Figure 2.

It was clarified based on the FCM analysis result that the ph01207 gene product of the N-terminal epitope-tag fusion type expresses on the cell membrane.

Among the clones for which expression of HA-tag fusion protein was observed, a cell line denominated as HA-ph01207#10-6 was deposited with the International Patent Organism Depositary of the National Institute of Advanced Industrial Science and Technology (Japan) on August 19, 2004 under Accession No. FERM BP-10101. The existence of this cell line was confirmed by experiment at the International Patent Organism Depositary on September 22, 2004. The HA-ph01207#10-6 cell line is a cell line that was established by transfecting into CHO-K1 cell line a vector that expresses, as an N-terminal HA-tag fusion protein, DNA consisting of a base sequence lacking a segment predicted to encode a signal sequence (20 amino acid residues from the N terminus of the amino acid sequence represented by SEQ ID NO: 2) among the open reading frame (ORF) of the DNA consisting of the base sequence represented by SEQ ID NO: 1, and it stably expresses the N-terminal HA-tag fusion protein.

### Example 3

### (Functional analysis of ph01207 gene product)

Functional analysis of the ph01207 gene product was carried out by determining the cell response when a ligand was added to *Xenopus laevis* oocyte that expressed ph01207. Determination of the cell response was carried out using six individuals each of control oocyte (oocyte into which the gene was not introduced) and oocyte that expressed the gene (ph01207 cDNA clone), by measuring variations in the membrane potential of the oocyte when a ligand was added thereto. Culture supernatant of the HeLa cell line in which expression of ph01207 was observed was used as the ligand. The culture supernatant was prepared by culturing HeLa cells of a cell count of 1.2 × 10⁶ in DMEM containing 10% fetal calf serum for two days, after which the culture supernatant was recovered and filtered with a filter (0.45 µm). Two kinds of culture supernatant with different lots prepared in the same manner were used as the ligand (hereunder, referred to as ligand sample 1 and 2).

The Ligand sample 1 or 2 was added to the oocyte that expressed the ph01207 gene and the control oocyte to determine membrane potential variations. Evaluation was carried out using variations in current amount and waveforms showing variations in current amount. When a variation in the current amount was 0.2 µA or more and a waveform of a GPCR-specific pattern was observed, it was judged that a response to ligand stimulation was generated. The phrase "GPCR-specific pattern" refers to the pattern of waveform 1 shown in Figure 3. The patterns of waveform 2-4 shown in Figure 3 are waveform patterns produced by artificial elements such as the influence of some kind of component such as a solvent, or a high concentration ligand, and thereby it was judged that a response generated was not against ligand stimulation. Further, when the patterns shown in waveform 5 and 6 were observed, it was judged that a response to ligand stimulation was not generated.

As a result, variations in the current amount were observed in only the ph01207 expression cells (Table 1 and Table 2). The characters "ND" in Table 2 indicate that the current variation amount was less than 0.2 µA and a response was not observed. As shown in Table 1, a response to ligand stimulation was observed in all six samples of the ph01207 expression cell lines. In contrast, there was completely no response to the ligand in the control cells (Table 2). The inventors therefore considered that the response of the ph01207 expression cells produced by ligand stimulation is a specific response to the expressed receptor. A similar result was obtained when using either of the ligand samples 1 and 2.

**Table 1**

| | ph01207 Expressing Oocyte | | | | | | | | |
|---|---|---|---|---|---|---|---|---|---|
| Ligand sample | Rate of response | Representative Waveform | Mean amount of current variation ± S.D. | Amount of current variation (µA) | | | | | |
| | | | | 1 | 2 | 3 | 4 | 5 | 6 |
| 1 | 6/6 | GPCR | 1.21±0.54 | 1.33 | 1.81 | 1.30 | 0.50 | 0.63 | 1.70 |
| 2 | 6/6 | GPCR | 0.48±0.24 | 0.66 | 0.24 | 0.29 | 0.58 | 0.82 | 0.27 |

**Table 2**

| | Control Oocyte | | | | | | | | |
|---|---|---|---|---|---|---|---|---|---|
| Ligand sample | Rate of response | Representative Waveform | Mean amount of current variation ± S.D. | Amount of current variation (µA) | | | | | |
| | | | | 1 | 2 | 3 | 4 | 5 | 6 |
| 1 | 0/6 | ND | | ND | ND | ND | ND | ND | ND |
| 2 | 0/6 | ND | | ND | ND | ND | ND | ND | ND |

As described above, because a cell response to ligand stimulation was produced by expression of the ph01207 gene, the inventors considered that the ph01207 gene product is a GPCR having a function that activates the intracellular signal transduction pathway in response to a ligand.

### Example 4

### (Analysis of protein interaction of ph01207 gene product)

Analysis of protein interaction of the ph01207 gene product was examined using the yeast two hybrid system.

Based on ph01207 sequence information and hBAI2 sequence information, bait was set respectively in the N-terminal region (4 places) and the C-terminal region (2 places) of the ph01207 gene product, and the N-terminal region (region lacking 55 amino acid residues in ph01207) and the C-terminal region (region having an insertion of one amino acid residue in ph01207) of the hBAI2 gene product, and screening was performed for a cDNA library (derived from brain, hippocampus, breast cancer and prostatic cancer, heart and skeletal muscle) that was selected according to a report (Non-Patent Literature 1) regarding the distribution of hBAI2 expression.

When using either the bait designed in the C-terminal region of the ph01207 gene product (having an insertion of one amino acid residue corresponding to position 1406 in the amino acid sequence represented by SEQ ID NO: 2) or the C-terminal region of the hBAI2 gene product (without insertion of the amino acid residue), the MAGUK family proteins DLG2, DLG3, DLG4, AIP1, MAGI3 and the like were obtained as prey. In addition, HOMER2, Citron, SYNE-1, KIF5A and KIFAP3 were obtained as prey that is specific to the hBAI2 gene product.

Although only BAT1 (HLA-B associated transcript 3) was obtained as prey for the bait designed in the N-terminal region (having deleted 55 amino acid residues) of the ph01207 gene product, KCNN2 (potassium intermediate/small conductance calcium-activated channel, subfamily N, member 2) and the like were obtained for the bait designed in the N-terminal region (without deletion of the amino acid residues) of the hBAI2 gene product.

It was thus found that, with the exception of MAGUK family proteins, there is a difference in the interacting proteins detected by the yeast two hybrid system for the ph01207 gene product and the hBAI2 gene product.

The ph01207 gene product and the hBAI2 gene product interact with MAGUK family proteins in their respective C-terminal regions. MAGUK family proteins are present in cytoplasm and bind with membrane proteins such as receptors or ion channels that are present in the cell membrane to participate in signal transduction from these membrane proteins. The inventors therefore considered that the ph01207 gene product and the hBAI2 gene product are functional membrane protein receptors that participate in intracellular signal transduction through MAGUK family proteins.

### Example 5

### (Determination of change in intracellular calcium concentration in ph01207 expression cell line caused by CCK 8)

Changes in intracellular calcium (Ca²⁺) concentrations caused by CCK-8S (SEQ ID NO: 14), CCK-8NS and CCK-4 were determined using the ph01207 expression cell lines prepared in Example 2. The cell lines prepared in Example 2 are 8 lines in which the protein encoded by ph01207 expresses as a FLAG-tag fusion protein and 4 lines in which the protein encoded by ph01207 expresses as a HA-tag fusion protein. Among these, in this example HA-ph01207# 10-6 cell line was used as a line obtained by cellular cloning of one cell line that stably expresses the protein encoded by ph01207 as a HA-tag fusion protein. Although CCK-8NS is a CCK octapeptide consisting of the same amino acid sequence as CCK-8S (SEQ ID NO: 14), the seventh tyrosine residue from the C-terminal thereof is not sulfated. CCK-4 is a tetrapeptide consisting of the amino acid residues from the C-terminus to the fourth amino acid residue of CCK-8S (SEQ ID NO: 14).

A specific method for measuring a change in intracellular Ca²⁺ concentration in the HA-ph01207# 10-6 cell line produced by CCK-8S (SEQ ID NO: 14) and the results thereof are described below. HA-ph01207#10-6 cell line was seeded in 96-well plates (plates with a black wall surface and transparent bottom) at a cell count of 2×10⁴/100 µL medium/well and cultured at 37°C in the presence of 5% CO₂. DMEM/F12 (Gibco) containing 10% fetal calf serum (Moregate BioTech) was used as medium. The next day, 50 µL of medium was extracted from each well, and 50 µL of loading buffer was added each well and allowed to react at room temperature for 1.5 h. The loading buffer was prepared by dissolving component A of the FLIPR Calcium 3 Assay Kit (Molecular Devices Corp.) with a solution in which 0.1 mL of 500 mM probenecid (Sigma) was added to 9.9 mL of component B. After reaction, variations with time in the fluorescence intensity upon addition of CCK-8S (Peptide Institute, Osaka, Japan) were measured for each well using FLEXstation (Molecular Devices Corp.). Measurement was also performed in a similar manner for CCK-8NS (Peptide Institute, Osaka, Japan) and CCK-4 (Peptide Institute). For CCK-8S (SEQ ID NO: 14), 0.52 mg thereof was dissolved with 4.5 mL of 1% NaHCO₃ (Wako) to prepare a 0.1 mM concentration solution. For CCK-8NS, after dissolving 0.53 mg thereof with 0.50 mL of dimethylsulfoxide (DMSO, Sigma), 4.50 mL of redistilled water was added thereto to prepare a 0.1 mM concentration solution. For CCK-4, after dissolving 0.54 mg thereof with 0.46 mL of DMSO, 4.09 mL of redistilled water was added thereto to prepare a 0.2 mM concentration solution. CCK-8S (SEQ ID NO: 14), CCK-8NS and CCK-4 were each diluted with phosphate buffered saline (PBS) to make a 5 nM solution, and measurement was performed after adding 25 µL (final concentration 1nM) of each. As a positive control that induces an increase in intracellular Ca²⁺ concentration, A23187 (Calbiochem, CA) was used at a final concentration of 10 µM. As a negative control, CHO-K1 cell line that was used as a host in production of the HA-ph01207#10-6 cell line was used. Since the ph01207 expression vector was not transfected into the CHO-K1 cell line, the ph01207 gene product was not expressed therein.

The results showed that the intracellular Ca²⁺ concentration of the HA-ph01207#10-6 cell line that was stimulated by CCK-8S (SEQ ID NO: 14) (Figure 4-A) rose in comparison to the HA-ph01207#10-6 cell line that was not stimulated (Figure 4-E). However, a rise in the intracellular Ca²⁺ concentration of the HA-ph01207#10-6 cell line was not observed in the cells stimulated by CCK-8NS or CCK-4 (Figure 4-B and Figure 4-C). Further, a rise in the intracellular Ca²⁺ concentration of the HA-ph01207#10-6 cell line stimulated by A23187 was observed (Figure 4-D).

The level of increase in the intracellular Ca²⁺ concentration of the HA-ph01207#10-6 cell line stimulated by 1 nM CCK-8S (SEQ ID NO: 14) (Figure 4-A) was roughly equal to level of increase in the intracellular Ca²⁺ concentration produced by A23187 that was used as a positive control (Figure 4-D).

In contrast, the CHO-K1 cell line showed no increase in intracellular Ca²⁺ concentration when stimulated with either CCK-8S (SEQ ID NO: 14), CCK-8NS or CCK-4 (Figure 4-A, Figure 4-B and Figure 4-C). However, an increase in the intracellular Ca²⁺ concentration of CHO-K1 cells upon stimulation with A23187 was observed (Figure 4-D).

From these results it was clarified that the HA-ph01207#10-6 cell line responds specifically to CCK-8S (SEQ ID NO: 14). It was also clarified that the protein encoded by ph01207 that was expressed in the HA-ph01207#10-6 cell line is involved in the increase in the intracellular Ca²⁺ concentration in the cell line caused by CCK-8S (SEQ ID NO: 14). The inventors therefore considered that CCK-8S (SEQ ID NO: 14) is a ligand of the ph01207 gene product.

The HA-ph01207#10-6 cell line also exhibited a sufficiently high biological response to 1 nM CCK-8S (SEQ ID NO: 14). Since CCK-8S (SEQ ID NO: 14) of a low concentration of 1 nM induced a biological response in the HA-ph01207#10-6 cell line, the inventors considered that CCK-8 actually induces a cellular biological response through the protein encoded by ph01207 *in vivo.* That it, the inventors considered that CCK-8S (SEQ ID NO: 14) is one of the *in vivo* ligands of ph01207 that is predicted to be a GPCR.

### INDUSTRIAL APPLICABILITY

According to the present invention there can be provided a protein derived from a novel functional membrane protein receptor that has a seven-span transmembrane domain which is considered a GPCR, and a DNA encoding the protein. The present protein is expressed on the cell membrane when expressed by a cell, and activates intracellular signal transduction by ligand stimulation to induce a cell response.

The present invention enables elucidation of signal transduction pathways and cell functions in which the present protein participates and the regulation thereof, as well as diagnosis, prevention and/or treatment of diseases attributable to an abnormality in the present protein and/or the DNA (for example, cerebral contusion or cerebral tumor) or tumor disease.

Further, CCK-8S (SEQ ID NO: 14) was found as an in vivo ligand of a functional membrane protein receptor according to the present invention. CCK-8S is a bioactive peptide that is involved in neurologic functions such as memory. Accordingly, by use of an agonist of the functional membrane protein receptor according to the present invention it is possible to carry out prevention and/or treatment of a disease accompanying a disorder of a neurologic function such as memory, for example, Alzheimer's disease.

Thus, the present invention is a useful substance that can make a contribution in a broad range of fields from basic science to pharmaceutical development.

### SEQUENCE TABLE FREE TEXT

SEQ ID NO: 1: DNA encoding a novel functional membrane protein receptor
SEQ ID NO: 2: Protein encoded by the DNA consisting of the base sequence of SEQ ID NO: 1
SEQ ID NO: 2: (297) : (350) TSP-I domain
SEQ ID NO: 2: (352) : (405) TSP-I domain
SEQ ID NO: 2: (408) : (461) TSP-I domain
SEQ ID NO: 2: (870) : (890) transmembrane domain
SEQ ID NO: 2: (899) : (919) transmembrane domain
SEQ ID NO: 2: (928) : (948) transmembrane domain
SEQ ID NO: 2: (970) : (990) transmembrane domain
SEQ ID NO: 2: (1012) : (1032) transmembrane domain
SEQ ID NO: 2: (1087) : (1107) transmembrane domain
SEQ ID NO: 2: (1114) : (1134) transmembrane domain
SEQ ID NO: 3: Partial amino acid sequence presented in each c-terminal region of a peptide consisting of the amino acid sequence of SEQ ID NO: 2, hBAI1 and hBAI2
SEQ ID NO: 4: Designed oligonucleotide for use as a primer
SEQ ID NO: 5: Designed oligonucleotide for use as a primer
SEQ ID NO: 6: Designed oligonucleotide for use as a primer
SEQ ID NO: 7: Designed oligonucleotide for use as a primer
SEQ ID NO: 8: Designed oligonucleotide for use as a primer
SEQ ID NO: 9: Designed oligonucleotide for use as a primer
SEQ ID NO: 10: Designed oligonucleotide for use as a primer
SEQ ID NO: 11: Designed oligonucleotide for use as a primer
SEQ ID NO: 12: Synthesized oligonucleotide
SEQ ID NO: 13: Synthesized oligonucleotide
SEQ ID NO: 14: CCK-8S
SEQ ID NO: 14: (2) (2) sulfated

## Claims

1. A method of identifying a compound that inhibits or promotes a function of a protein, encoded by DNA which is selected from the following group:
(i) a DNA consisting of a base sequence represented by SEQ ID NO: 1 of the sequence listing or a complementary strand thereof;
(ii) a DNA having a homology of at least 70% with the base sequence of the DNA of (i) and encoding a protein having a function that increases an intracellular calcium concentration or a function that generates a change in a cell membrane potential, or a complementary strand thereof;
(iii) a DNA comprising a base sequence of the DNA of (i) but one to one hundred nucleotides of which are deleted or substituted, or to which one to one hundred nucleotides are added, and encoding a protein having a function that increases an intracellular calcium concentration or a function that generates a change in a cell membrane potential, or a complementary strand thereof; and
(iv) a DNA hybridizing under stringent conditions with the DNA of (i) and encoding a protein having a function that increases an intracellular calcium concentration or a function that generates a change in a cell membrane potential, or a complementary strand thereof;
comprising inducing the function of the protein by the ligand of said protein which is:
(1) cholecystokinin octapeptide sulfated form of SEQ ID NO: 14 (CCK-8S), or
(2) a peptide comprising an amino acid sequence of CCK-8S but one amino acid of which is substituted with a homologous amino acid except for a sulfated tyrosine residue at the seventh position from the C-terminus of the peptide, and having a function that induces an increase of an intracellular calcium concentration or a change in a cell membrane potential in a cell expressing the DNA,
and wherein the method comprises using at least one member of the group consisting of the DNA, a recombinant vector containing the DNA, a transformant that was introduced with the recombinant vector, the HA-ph01207#10-6 cell line deposited under the Accession No. FERM BP-10101, a protein encoded by the DNA, and an antibody that immunologically recognizes said protein;
and, determining whether the test compound inhibits or promotes the function of said protein induced by said ligand, wherein said function of said protein is a function that increases an intracellular calcium concentration or wherein said function of said protein is a function that generates a change in a membrane potential.

2. The method according to claim 1, wherein the method comprises
(a) allowing said transformant or said cell line and a test compound to coexist under a condition that enables interaction of the transformant or the cell line with the test compound,
(b) introducing a system that measures said function of said protein that is expressed on a cell membrane of the transformant or the cell line, and
(c) determining whether or not the test compound inhibits or promotes the function of the protein by detecting the existence or non-existence of, or a change in, the function of the protein.

3. The method according to claim 1, wherein said function of said protein is a function that increases an intracellular calcium concentration and wherein the method comprises
(a) allowing said transformant or said cell line and a test compound to coexist under a condition that enables interaction of the transformant or the cell line with the test compound,
(b) measuring an intracellular calcium concentration of the transformant or the cell line, and
(c) determining whether or not the test compound inhibits or promotes the function of the protein by detecting a change in the intracellular calcium concentration of the transformant or the cell line.

4. The method according to claim 1, wherein said function of said protein is a function that generates a change in a membrane potential, and wherein the method comprises
(a) allowing said transformant or said cell line and a test compound to coexist under a condition that enables interaction of the transformant or the cell line with the test compound,
(b) measuring a membrane potential of the transformant or the cell line, and
(c) determining whether or not the test compound inhibits or promotes the function of the protein by detecting a change in the membrane potential of the transformant or the cell line.

5. A method of identifying a compound that inhibits or promotes binding of a protein, encoded by DNA which is selected from the following group:
(i) a DNA consisting of a base sequence represented by SEQ ID NO: 1 of the sequence listing or a complementary strand thereof;
(ii) a DNA having a homology of at least 70% with the base sequence of the DNA of (i) and encoding a protein having a function that increases an intracellular calcium concentration or a function that generates a change in a cell membrane potential, or a complementary strand thereof;
(iii) a DNA comprising a base sequence of the DNA of (i) but one to one hundred nucleotides of which are deleted or substituted, or to which one to one hundred nucleotides are added, and encoding a protein having a function that increases an intracellular calcium concentration or a function that generates a change in a cell membrane potential, or a complementary strand thereof; and
(iv) a DNA hybridizing under stringent conditions with the DNA of (i) and encoding a protein having a function that increases an intracellular calcium concentration or a function that generates a change in a cell membrane potential, or a complementary strand thereof;
to a ligand of said protein wherein the ligand is:
(1) cholecystokinin octapeptide sulfated form of SEQ ID NO: 14, or
(2) a peptide comprising an amino acid sequence of CCK-8S but one amino acid of which is substituted with a homologous amino acid except for a sulfated tyrosine residue at the seventh position from the C-terminus of the peptide, and having a function that induces an increase of an intracellular calcium concentration or a change in a cell membrane potential in a cell expressing the DNA;
and wherein said protein and said ligand of the protein are reacted in the presence and absence of the compound to measure binding between the protein and the ligand.

6. Method according to claim 5, wherein the protein is expressed in a cell containing DNA encoding the protein or in a transformant such as the HA-ph01207#10-6 cell line deposited under the Accession No. FERM BP-10101.

7. Method according to claim 5, wherein the binding is measured by measuring a change in cell membrane potential or a change in intracellular calcium concentration caused by said ligand in the presence and absence of the compound.

## Patentansprüche

1. Verfahren zum Identifizieren einer Verbindung, die eine Funktion eines Proteins inhibiert oder befördert, wobei das Protein durch eine DNA kodiert wird, die aus der folgenden Gruppe ausgewählt ist:
(i) eine DNA, die aus einer durch SEQ ID NO: 1 des Sequenzprotokolls dargestellten Basensequenz besteht, oder ein komplementärer Strang derselben;
(ii) eine DNA, die eine Homologie von wenigstens 70% zur Basensequenz der DNA nach (i) aufweist und die ein Protein kodiert, welches eine Funktion, die eine intrazelluläre Calciumkonzentration erhöht, oder eine Funktion, die eine Änderung in einem Zellmembranpotential erzeugt, aufweist, oder ein komplementärer Strang derselben;
(iii) eine DNA, die eine Basensequenz der DNA nach (i) umfasst, wobei aber ein bis 100 Nukleotide derselben deletiert oder substituiert sind oder zu der ein bis 100 Nukleotide hinzugefügt sind, und die ein Protein kodiert, das eine Funktion, die eine intrazelluläre Calciumkonzentration erhöht, oder eine Funktion, die eine Änderung in einem Zellmembranpotential erzeugt, aufweist, oder ein komplementärer Strang derselben; und
(iv) eine DNA, die unter stringenten Bedingungen mit der DNA nach (i) hybridisiert und die ein Protein kodiert, das eine Funktion, die eine intrazelluläre Calciumkonzentration erhöht, oder eine Funktion, die eine Änderung in einem Zellmembranpotential erzeugt, aufweist, oder ein komplementärer Strang derselben;
umfassend das Induzieren der Funktion des Proteins durch den Liganden des Proteins, welcher ist:
(1) die sulfatierte Form des Cholecystokinin-Octapeptids nach SEQ ID NO: 14 (CCK-8S) oder
(2) ein Peptid, das eine Aminosäuresequenz von CCK-8S umfasst, bei der aber eine Aminosäure durch eine homologe Aminosäure ausgetauscht ist mit Ausnahme eines sulfatierten Tyrosin-Rests an der siebten Position vom C-Terminus des Peptids, und das eine Funktion aufweist, die eine Erhöhung einer intrazellulären Calciumkonzentration oder eine Veränderung in einem Zellmembranpotential in einer Zelle induziert, die die DNA exprimiert,
und wobei das Verfahren die Verwendung wenigstens eines Mitglieds aus der Gruppe umfasst, die aus der DNA, einem rekombinanten Vektor, der die DNA enthält, einer Transformante, in die der rekombinante Vektor eingeführt wurde, der Zelllinie HA-ph01207#10-6, die unter der Zugangsnummer FERM BP-10101 hinterlegt worden ist, einem Protein, das durch die DNA kodiert wird, und einem Antikörper, der das Protein immunologisch erkennt, besteht;
und die Bestimmung, ob die Testverbindung die Funktion des durch den Liganden induzierten Proteins inhibiert oder befördert, wobei die Funktion des Proteins eine Funktion ist, die eine intrazelluläre Calciumkonzentration erhöht, oder wobei die Funktion des Proteins eine Funktion ist, die eine Änderung in einem Membranpotential erzeugt.

2. Verfahren gemäß Anspruch 1, wobei das Verfahren umfasst
(a) die Transformante oder die Zelllinie und eine Testverbindung unter einer Bedingung koexistieren lassen, die Interaktion der Transformante oder der Zelllinie mit der Testverbindung ermöglicht,
(b) Einbringen eines Systems, das die Funktion des Proteins misst, das auf einer Zellmembran der Transformante oder der Zelllinie exprimiert wird, und
(c) Bestimmen, ob die Testverbindung die Funktion des Proteins inhibiert oder nicht oder befördert oder nicht, indem das Vorhandensein oder das Nichtvorhandensein der Funktion oder eine Veränderung in der Funktion des Proteins bestimmt wird.

3. Verfahren gemäß Anspruch 1, wobei die Funktion des Proteins eine Funktion ist, die eine intrazelluläre Calciumkonzentration erhöht, und wobei das Verfahren umfasst
(a) die Transformante oder die Zelllinie und eine Testverbindung unter einer Bedingung koexistieren lassen, die Interaktion der Transformante oder der Zelllinie mit der Testverbindung ermöglicht,
(b) Messen einer intrazellulären Calciumkonzentration der Transformante oder der Zelllinie, und
(c) Bestimmen, ob die Testverbindung die Funktion des Proteins inhibiert oder nicht oder befördert oder nicht, indem eine Änderung in der intrazellulären Calciumkonzentration der Transformante oder der Zelllinie bestimmt wird.

4. Verfahren gemäß Anspruch 1, wobei die Funktion des Proteins eine Funktion ist, die eine Änderung in einem Membranpotential erzeugt, und wobei das Verfahren umfasst
(a) die Transformante oder die Zelllinie und eine Testverbindung unter einer Bedingung koexistieren lassen, die Interaktion der Transformante oder der Zelllinie mit der Testverbindung ermöglicht,
(b) Messen eines Membranpotentials der Transformante oder der Zelllinie und
(c) Bestimmen, ob die Testverbindung die Funktion des Proteins inhibiert oder nicht oder befördert oder nicht, indem eine Änderung in dem Membranpotential der Transformante oder der Zelllinie bestimmt wird.

5. Verfahren zum Identifizieren einer Verbindung, die die Bindung eines Proteins an einen Liganden des Proteins inhibiert oder befördert, wobei das Protein durch eine DNA kodiert wird, die aus der folgenden Gruppe ausgewählt ist:
(i) eine DNA, die aus einer durch SEQ ID NO: 1 des Sequenzprotokolls dargestellten Basensequenz besteht, oder ein komplementärer Strang derselben;
(ii) eine DNA, die eine Homologie von wenigstens 70% zur Basensequenz der DNA nach (i) aufweist und die ein Protein kodiert, welches eine Funktion, die eine intrazelluläre Calciumkonzentration erhöht, oder eine Funktion, die eine Änderung in einem Zellmembranpotential erzeugt, aufweist, oder ein komplementärer Strang derselben;
(iii) eine DNA, die eine Basensequenz der DNA nach (i) umfasst, wobei aber ein bis 100 Nukleotide derselben deletiert oder substituiert sind oder zu der ein bis 100 Nukleotide hinzugefügt sind, und die ein Protein kodiert, das eine Funktion, die eine intrazelluläre Calciumkonzentration erhöht, oder eine Funktion, die eine Änderung in einem Zellmembranpotential erzeugt, aufweist, oder ein komplementärer Strang derselben; und
(iv) eine DNA, die unter stringenten Bedingungen mit der DNA nach (i) hybridisiert und die ein Protein kodiert, das eine Funktion, die eine intrazelluläre Calciumkonzentration erhöht, oder eine Funktion, die eine Änderung in einem Zellmembranpotential erzeugt, aufweist, oder ein komplementärer Strang derselben;
wobei der Ligand ist:
(1) die sulfatierte Form des Cholecystokinin-Octapeptids nach SEQ ID NO: 14 (CCK-8S) oder
(2) ein Peptid, das eine Aminosäuresequenz von CCK-8S umfasst, bei der aber eine Aminosäure durch eine homologe Aminosäure ausgetauscht ist mit Ausnahme eines sulfatierten Tyrosin-Rests an der siebten Position vom C-Terminus des Peptids, und das eine Funktion aufweist, die eine Erhöhung einer intrazellulären Calciumkonzentration oder eine Veränderung in einem Zellmembranpotential in einer Zelle induziert, die die DNA exprimiert,
und wobei das Protein und der Ligand des Proteins in Anwesenheit und in Abwesenheit der Verbindung umgesetzt werden, um die Bindung zwischen dem Protein und dem Liganden zu messen.

6. Verfahren gemäß Anspruch 5, wobei das Protein in einer Zelle, die DNA enthält, die das Protein kodiert, oder in einer Transformante, wie z.B. der Zelllinie HA-ph01207#10-6, die unter der Zugangsnummer FERM BP-10101 hinterlegt worden ist, exprimiert wird.

7. Verfahren gemäß Anspruch 5, wobei die Bindung gemessen wird, indem eine Veränderung im Zellmembranpotential oder eine Veränderung in intrazellulärer Calciumkonzentration, die durch den Liganden bei Anwesenheit oder Abwesenheit der Verbindung verursacht wird, gemessen wird.

## Revendications

1. Procédé pour l'identification d'un composé qui inhibe ou favorise une fonction d'une protéine, codée par un ADN qui est choisi dans le groupe suivant:
(i) un ADN consistant en une séquence de bases représentée par SEQ ID NO: 1 du listage de séquences, ou un brin complémentaire de celui-ci;
(ii) un ADN ayant une homologie d'au moins 70% avec la séquence de bases de l'ADN de (i) et codant pour une protéine ayant une fonction qui augmente une concentration en calcium intracellulaire ou une fonction qui génère une modification dans un potentiel de membrane cellulaire, ou un brin complémentaire de celui-ci;
(iii) un ADN comprenant une séquence de bases de l'ADN de (i) mais dont un à cent nucléotides sont délétés ou substitués, ou auquel un à cent nucléotides sont ajoutés, et codant pour une protéine ayant une fonction qui augmente une concentration en calcium intracellulaire ou une fonction qui génère une modification dans un potentiel de membrane cellulaire, ou un brin complémentaire de celui-ci; et
(iv) un ADN hybridant dans des conditions stringentes avec l'ADN de (i) et codant pour une protéine ayant une fonction qui augmente une concentration en calcium intracellulaire ou une fonction qui génère une modification dans un potentiel de membrane cellulaire, ou un brin complémentaire de celui-ci;
comprenant l'induction de la fonction de la protéine par le ligand de ladite protéine qui est:
(1) la forme octapeptide sulfaté de la cholécystokinine de SEQ ID NO:14 (CCK-8S), ou
(2) un peptide comprenant une séquence d'acides aminés de CCK-8S mais dont un acide aminé est substitué par un acide aminé homologue hormis pour un résidu tyrosine sulfaté sur la septième position à partir de l'extrémité C-terminale du peptide, et ayant une fonction qui induit une augmentation d'une concentration en calcium intracellulaire ou une modification dans un potentiel de membrane cellulaire dans une cellule exprimant l'ADN,
et tandis que le procédé comprend l'utilisation d'au moins un membre du groupe consistant en l'ADN, un vecteur recombinant contenant l'ADN, un transformant qui a été introduit avec le vecteur recombinant, la lignée cellulaire HA-phOl2O7#10-6 déposée sous le numéro d'accès FERM BP-10101, une protéine codée par l'ADN, et un anticorps qui reconnaît de manière immunologique ladite protéine; et la détermination de ce que le composé d'essai inhibe ou non ou favorise ou non la fonction de ladite protéine induite par ledit ligand, tandis que ladite fonction de ladite protéine est une fonction qui augmente une concentration en calcium intracellualire ou tandis que ladite fonction de ladite protéine est une fonction qui génère une modification dans un potentiel de membrane.

2. Procédé selon la revendication 1, dans lequel le procédé comprend
(a) le fait de laisser ledit transformant ou ladite lignée cellulaire et un composé d'essai coexister dans une condition qui permet l'interaction du transformant ou de la lignée cellulaire avec le composé d'essai,
(b) l'introduction d'un système qui mesure ladite fonction de ladite protéine qui est exprimée sur une membrane cellulaire du transformant ou de la lignée cellulaire, et
(c) la détermination de ce que le composé d'essai inhibe ou non ou favorise ou non la fonction de la protéine par détection de l'existence ou de la non-existence de, ou d'une modification dans, la fonction de la protéine.

3. Procédé selon la revendication 1, dans lequel ladite fonction de ladite protéine est une fonction qui augmente une concentration en calcium intracellulaire et dans lequel le procédé comprend
(a) le fait de laisser ledit transformant ou ladite lignée cellulaire et un composé d'essai coexister dans une condition qui permet l'interaction du transformant ou de la lignée cellulaire avec le composé d'essai,
(b) la mesure d'une concentration en calcium intracellulaire du transformant ou de la lignée cellulaire, et
(c) la détermination de ce que le composé d'essai inhibe ou non ou favorise ou non la fonction de la protéine par détection d'une modification dans la concentration de calcium intracellulaire du transformant ou de la lignée cellulaire.

4. Procédé selon la revendication 1, dans lequel ladite fonction de ladite protéine est une fonction qui génère une modification dans un potentiel de membrane, et tandis que le procédé comprend
(a) le fait de laisser ledit transformant ou ladite lignée cellulaire et un composé d'essai coexister dans une condition qui permet l'interaction du transformant ou de la lignée cellulaire avec le composé d'essai,
(b) la mesure d'un potentiel de membrane du transformant ou de la lignée cellulaire, et
(c) la détermination de ce que le composé d'essai inhibe ou non ou favorise ou non la fonction de la protéine par détection d'une modification dans le potentiel de membrane du transformant ou de la lignée cellulaire.

5. Procédé pour l'identification d'un composé qui inhibe ou favorise la liaison d'une protéine, codée par un ADN qui est choisi dans le groupe suivant:
(i) un ADN consistant en une séquence de bases représentée par SEQ ID NO: 1 du listage de séquences ou un brin complémentaire de celui-ci;
(ii) un ADN ayant une homologie d'au moins 70% avec la séquence de bases de l'ADN de (i) et codant pour une protéine ayant une fonction qui augmente une concentration en calcium intracellulaire ou une fonction qui génère une modification dans un potentiel de membrane cellulaire, ou un brin complémentaire de celui-ci;
(iii) un ADN comprenant une séquence de bases de l'ADN de (i) mais dont un à cent nucléotides sont délétés ou substitués, ou auquel un à cent nucléotides sont ajoutés, et codant pour une protéine ayant une fonction qui augmente une concentration en calcium intracellulaire ou une fonction qui génère une modification dans un potentiel de membrane cellulaire, ou un brin complémentaire de celui-ci; et
(iv) un ADN hybridant dans des conditions stringentes avec l'ADN de (i) et codant pour une protéine ayant une fonction qui augmente une concentration en calcium intracellulaire ou une fonction qui génère une modification dans un potentiel de membrane cellulaire, ou un brin complémentaire de celui-ci;
à un ligand de ladite protéine, tandis que le ligand est:
(1) la forme octapeptide sulfaté de la cholécystokinine de SEQ ID NO: 14, ou
(2) un peptide comprenant une séquence d'acides aminés de CCK-8S mais dont un acide aminé est substitué par un acide aminé homologue hormis pour un résidu tyrosine sulfaté sur la septième position à partir de l'extrémité C-terminale du peptide, et ayant une fonction qui induit une augmentation d'une concentration en calcium intracellulaire ou une modification dans un potentiel de membrane cellulaire dans une cellule exprimant l'ADN;
et tandis que ladite protéine et ledit ligand de la protéine sont mis à réagir en présence et en l'absence du composé pour mesurer la liaison entre la protéine et le ligand.

6. Procédé selon la revendication 5, dans lequel la protéine est exprimée dans une cellule contenant un ADN codant pour la protéine ou dans un transformant tel que la lignée cellulaire HA-ph0127#10-6 déposée sous le numéro d'accès FERM BP-10101.

7. Procédé selon la revendication 5, dans lequel la liaison est mesurée par mesure d'une modification dans le potentiel de membrane cellulaire ou une modification dans la concentration de calcium intracellulaire provoquée par ledit ligand en présence ou en l'absence du composé.
